# EUROPEAN PATENT APPLICATION

(11) **EP 3 712 268 A2**
(43) Date of publication of application: **23.09.2020**
(21) Application number: 18877963.1
(22) Date of filing: 16.11.2018
(51) Int. Cl.: C12N 15/113, C12N 9/22, C12N 15/10, C12N 15/90, C12N 5/0783, A61K 35/17, A61P 35/00

(54) **TRANSFORMED HUMAN CELL AND USE THEREOF**

(30) Priority: 16.11.2017 US 201762587068 P
(71) Applicant: Mogam Institute for Biomedical Research, Yongin-si, Gyeonggi-do 16924 (KR)
(72) Inventor: LIM, Ok Jae, Yongin-si Gyeonggi-do 16924 (KR); KIM, Mun Kyung, Yongin-si Gyeonggi-do 16924 (KR); LEE, Yun Jung, Yongin-si Gyeonggi-do 16924 (KR); LEE, Jee Won, Yongin-si Gyeonggi-do 16924 (KR); YANG, Woo Seok, Yongin-si Gyeonggi-do 16924 (KR); KIM, Yu Young, Yongin-si Gyeonggi-do 16924 (KR); KWON, Young Eun, Yongin-si Gyeonggi-do 16294 (KR); CHOE, Seung Hyon, Yongin-si Gyeonggi-do 16924 (KR)
(74) Representative: dompatent von Kreisler Selting Werner - Partnerschaft von Patent- und Rechtsanwälten mbB
(86) International application number: PCT/KR2018/014112
(87) International publication number: WO 2019/098759

(57) **Abstract**

The present invention relates to a transformed human cell and a use thereof and, more particularly, to a cell transformed with a gene for coding an MHC I cell membrane receptor and an MHC II cell membrane receptor by using a gene expression suppressing system using a guide RNA, and a use thereof. Such a transformed cell can effectively exhibit the therapeutic effect of cells even *in vivo,* and cannot be removed by an *in vivo* immune response. Therefore, it is expected that a composition comprising the immunocyte as an active ingredient can be usefully used for the treatment of cancer, infectious diseases, degenerative diseases or immunological diseases.

## Description

### Technical Field

The present invention relates to a transformed human cell and a use thereof, and more particularly, to a human cell transformed through a guide RNA and a use thereof

### Background Art

As a method for treating cancer or an infectious disease, immunotherapies using the patient's immune function are attracting attention. Immunotherapies mean treatment methods for diseases through interaction of immune cells such as NK cells, T cells, dendritic cells, and the like. Among these, immunotherapies are emerging which use genetically modified T cells expressing a chimeric antigen receptor specific for an antigen. In addition, it has been reported that NK cells, which are allowed to have high cytotoxicity by being activated *ex vivo,* exhibit an excellent therapeutic effect on blood cancer such as leukemia (Blood Cells Molecules & Disease, 33: p261-266, 2004).

Meanwhile, despite possibility of immune cells as a therapeutic agent for cancer or an infectious disease as mentioned above, immune cells present in a patient's body are remarkably lower, in terms of function and number, as compared with those in healthy individuals. Therefore, it is more effective to utilize transplantation of allogeneic immune cells than to use autologous immune cells. However, in a case where allogeneic immune cells are transplanted, several problems may occur, such as transplant rejection, or immunological elimination caused by recognition of non-self *in vivo.* Accordingly, in order to overcome these drawbacks, there is a need for an alternative to making allogeneic immune cells into a cell banking while allowing the allogeneic immune cells to be recognized as self.

### Disclosure of Invention

### Technical Problem

In order to solve the above-mentioned problems, the present inventors have synthesized guide RNAs that target a gene encoding MHC I cell membrane receptor and a gene encoding MHC II cell membrane receptor in a cell. In addition, the present inventors have prepared a cell, in which expression of MHC I cell membrane receptor and MHC II cell membrane receptor is inhibited, using a composition for inhibiting gene expression which comprises, as active ingredients, the guide RNA and an RNA-guided endonuclease, wherein HLA-E may be introduced thereto so that *in vivo* immunological elimination to the cell is prevented.

Accordingly, an object of the present invention is to provide guide RNAs that target a gene encoding MHC I cell membrane receptor and a gene encoding MHC II cell membrane receptor, and to provide a cell transformed using the guide RNA.

### Solution to Problem

In order to achieve the above object, the present invention provides a guide RNA that complementarily binds to a nucleic acid sequence encoding β2-microglobulin (B2M), the guide RNA comprising any one nucleic acid sequence selected from the group consisting of SEQ ID NO: 1, SEQ ID NO: 6, SEQ ID NO: 17, and SEQ ID NO: 26; a guide RNA that complementarily binds to a nucleic acid sequence encoding HLA-DQ, the guide RNA comprising any one nucleic acid sequence selected from the group consisting of SEQ ID NO: 64, SEQ ID NO: 65, SEQ ID NO: 87, and SEQ ID NO: 90; a guide RNA that complementarily binds to a nucleic acid sequence encoding HLA-DP, the guide RNA comprising the nucleic acid sequence of SEQ ID NO: 123 or SEQ ID NO: 129; and a guide RNA that complementarily binds to a nucleic acid sequence encoding HLA-DR, the guide RNA comprising any one nucleic acid sequence selected from the group consisting of SEQ ID NO: 186, SEQ ID NO: 188, and SEQ ID NO: 225.

In addition, the present invention provides a composition for inhibiting gene expression comprising as active ingredients a guide RNA or a nucleotide sequence encoding the guide RNA, and an RNA-guided endonuclease or a nucleotide sequence encoding the RNA-guided endonuclease.

In addition, the present invention provides a transformed cell in which expression of MHC I cell membrane receptor and MHC II cell membrane receptor is inhibited.

In addition, the present invention provides a pharmaceutical composition for treating cancer, an infectious disease, a degenerative disease, a hereditary disease, or an immune disease, comprising the transformed cell as an active ingredient; and a method for treating cancer, an infectious disease, a degenerative disease, a hereditary disease, or an immune disease, comprising administering the composition to a subject.

In addition, the present invention provides a use of a transformed cell for treating cancer, an infectious disease, a degenerative disease, a hereditary disease, or an immune disease, wherein expression of MHC I cell membrane receptor and MHC II cell membrane receptor is inhibited in the transformed cell, and the transformed cell expresses a peptide antigen, such as G-peptide, bound to a modified MHC I cell membrane receptor on the cell membrane surface.

### Advantageous Effects of Invention

It is possible to prepare a cell in which a gene encoding MHC I cell membrane receptor and a gene encoding MHC II cell membrane receptor are modified, by using a gene expression inhibition system using a guide RNA according to the present invention. In addition, it is possible to additionally introduce, into the cell, HLA-E to which a peptide antigen such as G-peptide is bound. A cell transformed as described above can effectively show its therapeutic efficacy even *in vivo,* and is not eliminated by an *in vivo* immune response. Therefore, it is expected that a composition comprising the cell as an active ingredient can be usefully used for the treatment of cancer, an infectious disease, a degenerative disease, a hereditary disease, or an immune disease.

### Brief Description of Drawings

Fig. 1 illustrates results obtained by analyzing, with flow cytometry, HLA-ABC negative cells in cells prepared by using a B2M-targeted gRNA.
Fig. 2 illustrates results obtained by analyzing, with flow cytometry, HLA-DR negative cells in cells prepared by using an HLA-DRA-targeted gRNA.
Fig. 3 illustrates results obtained by analyzing, with flow cytometry, HLA-DQ negative cells in cells prepared by using an HLA-DQA-targeted gRNA.
Fig. 4 illustrates results obtained by analyzing, with flow cytometry, HLA-DP negative cells in cells prepared by using an HLA-DPA-targeted gRNA.
Fig. 5 illustrates production rates of HLA-ABC negative cell line depending on B2M-targeted gRNAs.
Fig. 6 illustrates production rates of HLA-DR negative cell line depending on DRA-targeted gRNAs.
Fig. 7 illustrates production rates of HLA-DQ negative cell line depending on DQA-targeted gRNAs.
Fig. 8 illustrates production rates of HLA-DP negative cell line depending on DPA-targeted gRNAs.
Fig. 9 illustrates mutation in a nucleic acid encoding B2M in cell lines prepared with B2M-targeted gRNAs.
Fig. 10 illustrates mutation in a nucleic acid encoding HLA-DRA in cell lines prepared with HLA-DRA-targeted gRNAs.
Fig. 11 illustrates mutation in a nucleic acid encoding HLA-DQA in cell lines prepared with HLA-DQA-targeted gRNAs.
Fig. 12 illustrates mutation in a nucleic acid encoding HLA-DPA in cell lines prepared with HLA-DPA-targeted gRNAs.
Fig. 13 illustrates HLA-I positive NK-92MI cell line and HLA-I negative NK-92MI cell line after cell separation.
Fig. 14 illustrates evaluation results for cell-killing capacity of the HLA-I positive NK-92MI cell line and the HLA-I negative NK-92MI cell line.
Fig. 15 illustrates results obtained by transforming CD4 T cells, CD8 T cells, and NK cells using gRNAs and then performing analysis with flow cytometry.
Fig. 16 illustrates deletion efficiency for targets in single gRNA-transformed cells and multiple gRNA-transformed cells.
Fig. 17 compares cell growth rate among single gRNA-transformed cells, multiple gRNA-transformed cells, and control group cells.
Fig. 18 compares cytokine production capacity between HLA-I positive T cells and HLA-I negative T cells.
Fig. 19 compares cytokine production capacity between HLA-I positive NK cells and HLA-I negative NK cells.
Fig. 20 illustrates evaluation results for cell-killing capacity of NK cells against HLA-I positive Raji cell line and HLA-I negative Raji cell line.
Fig. 21 illustrates a schematic diagram of HLA-E loaded with G-peptide and a structure of a protein for expressing the same.
Fig. 22 illustrates results obtained by analyzing HLA-E expressed in K562 cell line through transduction.
Fig. 23 illustrates evaluation results for cell-killing capacity of NK cells against K562 cell line (K562 G-B2M-HLA-E) expressing HLA-E and control group K562 cell line (K562).

### Best Mode for Carrying out the Invention

In an aspect of the present invention, there is provided a guide RNA that complementarily binds to a nucleic acid sequence encoding β2-microglobulin (B2M), the guide RNA comprising any one nucleic acid sequence selected from the group consisting of SEQ ID NO: 1, SEQ ID NO: 6, SEQ ID NO: 17, and SEQ ID NO: 26.

As used herein, the term "B2M" refers to β2-microglobulin protein that is a component of MHC I. B2M is essential for expression of MHC I cell membrane receptor on the cell surface; and when B2M is removed or modified, expression of the MHC I cell membrane receptor on the cell surface is difficult to occur. Thus, the function of the MHC I cell membrane receptor may be removed by modifying the gene of B2M.

The guide RNA that complementarily binds to a nucleic acid sequence encoding B2M may be any one selected from the group consisting of SEQ ID NOs: 1 to 58, and may specifically be any one selected from the group consisting of SEQ ID NO: 1, SEQ ID NO: 6, SEQ ID NO: 17, and SEQ ID NO: 26.

In addition, in an aspect of the present invention, there is provided a guide RNA that complementarily binds to a nucleic acid sequence encoding HLA-DQ, the guide RNA comprising any one nucleic acid sequence selected from the group consisting of SEQ ID NO: 64, SEQ ID NO: 65, SEQ ID NO: 87, and SEQ ID NO: 90.

As used herein, the term "HLA" refers to a human leukocyte antigen that is a product of MHC gene. HLA is composed of HLA I and HLA II. HLA I may include HLA-A, HLA-B, and HLA-C; and HLA II may include HLA-DQ, HLA-DP, and HLA-DR.

As used herein, the term "HLA-DQ" refers to an αβ heterodimer constituting MHC II. DQ consists of HLA-DQA1 and HLA-DQB1. The α subunit is encoded by HLA-DQA1 gene, and the β subunit is encoded by HLA-DQB1 gene. Expression of MHC II cell membrane receptor may be inhibited by modifying the gene of DQ.

The guide RNA that complementarily binds to a nucleic acid sequence encoding DQ may be any one selected from the group consisting of SEQ ID NOs: 59 to 116, and may specifically be any one selected from the group consisting of SEQ ID NO: 64, SEQ ID NO: 65, SEQ ID NO: 87, and SEQ ID NO: 90.

In another aspect of the present invention, there is provided a guide RNA that complementarily binds to a nucleic acid sequence encoding HLA-DP, the guide RNA comprising the nucleic acid sequence of SEQ ID NO: 123 or SEQ ID NO: 129.

As used herein, the term "HLA-DP" refers to an encoded MHC II cell surface receptor that consists of DPα subunit and DPβ subunit. DPα is encoded by HLA-DPA1, and DPβ is encoded by HLA-DPB1. Expression of MHC II cell membrane receptor may be inhibited by modifying the gene of DP.

The guide RNA that complementarily binds to a nucleic acid sequence encoding DP may be any one selected from the group consisting of SEQ ID NOs: 117 to 175, and may specifically be SEQ ID NO: 123 or SEQ ID NO: 129.

In addition, in yet another aspect of the present invention, there is provided a guide RNA that complementarily binds to a nucleic acid sequence encoding HLA-DR, the guide RNA comprising any one nucleic acid sequence selected from the group consisting of SEQ ID NO: 186, SEQ ID NO: 188, and SEQ ID NO: 225.

As used herein, the term "HLA-DR" refers to an MHC II cell surface receptor, specifically an αβ heterodimer that constitutes the MHC II cell surface receptor. Each subunit of HLA-DR contains two extracellular domains, a membrane-spanning domain and a cytoplasmic tail. Expression of MHC II cell membrane receptor may be inhibited by modifying the gene of DR.

The guide RNA that complementarily binds to a nucleic acid sequence encoding DR may be any one selected from the group consisting of SEQ ID NOs: 176 to 234, and may preferably be any one selected from the group consisting of SEQ ID NO: 186, SEQ ID NO: 188, and SEQ ID NO: 225.

As used herein, the term "guide RNA (gRNA)" refers to an RNA molecule that specifically recognizes a target DNA and forms a complex with a nuclease, thereby guiding the nuclease to the target DNA.

The guide RNA may be a guide RNA derived from a prokaryotic clustered regularly interspaced short palindromic repeats (CRISPR) system.

The guide RNA may contain a non-naturally occurring chimeric crRNA sequence, and the crRNA sequence may contain a variable targeting domain capable of hybridizing to a target sequence.

In addition, the guide RNA contains a complementary sequence for each of B2M, HLA-DQ, HLA-DP, and HLA-DR genes. After being delivered into a cell, the guide RNA is capable of recognizing the target sequence and forming a complex with an RNA-guided endonuclease.

In yet another aspect of the present invention, there is provided a composition for inhibiting gene expression, comprising as active ingredients, the guide RNA or a nucleotide sequence encoding the guide RNA, and an RNA-guided endonuclease or a nucleotide sequence encoding the RNA-guided endonuclease.

The RNA-guided endonuclease may be delivered in the form of mRNA or protein, or may be delivered to a target cell by transformation using a vector loaded with DNA encoding the same. When an endonuclease in the form of protein is used, the endonuclease may function as an RNP complex obtained by forming a complex with the guide RNA.

As used herein, the term "RNP complex" refers to a complex that comprises, as active ingredients, the guide RNA and the RNA-guided endonuclease, wherein the complex is capable of recognizing and binding to a target sequence, thereby selectively nicking or cleaving the target sequence. The RNA complex may be, for example, a Cas9-gRNA complex but is not limited thereto.

In an embodiment of the present invention, the RNA-guided endonuclease may be any one selected from the group consisting of Cas1, Cas1B, Cas2, Cas3, Cas4, Cas5, Cas6, Cas7, Cas8, Cas9, Cas10, Cas12a, Cas12b, Cas12c, Cas12d, Cas12e, Cas 13a, Cas 13b, Cas 13c, Cas 13d, Cpf1, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, and Csf4, and may specifically be Cas9.

In an aspect of the present invention, there is provided a transformed cell in which expression of MHC I cell membrane receptor and MHC II cell membrane receptor is inhibited.

As used herein, the term "expression inhibition" means modification on a nucleotide sequence which causes a decrease in the function of a target gene, and preferably means that expression of a target gene is made undetectable or the target gene is expressed to a meaningless level, due to such expression inhibition.

In an embodiment of the present invention, the transformed cell may express a peptide antigen on the cell membrane surface. Examples of the peptide antigen include, but are not limited to, signal peptides of HLA-A, HLA-B, HLA-C, and HLA-G, and the peptide antigen is specifically a signal peptide (G-peptide) of HLA-G. The peptide antigen may be bound to modified MHC I cell membrane receptor.

In an embodiment of the present invention, the modified MHC I cell membrane receptor has a structure in which HLA-E and B2M are linked. Specifically, the C-terminus of B2M may be linked, via a first linker, to the N-terminus of α1 of HLA-E and the C-terminus of G-peptide may be linked, via a second linker, to the N-terminus of B2M in the modified MHC I cell membrane receptor. The modified MHC I cell membrane receptor may have a structure in which HLA-G and B2M are linked.

In an embodiment of the present invention, G-peptide may have the sequence of SEQ ID NO: 236; HLA-E may have the sequence of SEQ ID NO: 240; B2M may have the sequence of SEQ ID NO: 237; and the first linker may be (G₄S)ₙ (n is an integer of 1 to 5) and may have the sequence of SEQ ID NO: 238 in an embodiment. The second linker may be (G₄S)ₙ (n is an integer of 2 to 6) and may have the sequence of SEQ ID NO: 241.

In an embodiment of the present invention, modification of a gene encoding the MHC I cell membrane receptor may be performed using the guide RNA (for example, SEQ ID NO: 1, SEQ ID NO: 6, SEQ ID NO: 17, or SEQ ID NO: 26) that complementarily binds to a nucleic acid sequence encoding B2M. Specifically, the modification of MHC I may be performed by single deletion using a single guide RNA.

In an embodiment of the present invention, modification of DQ, DP, and DR genes encoding the MHC II cell membrane receptor may be performed using the guide RNA (for example, SEQ ID NO: 64, SEQ ID NO: 65, SEQ ID NO: 87, or SEQ ID NO: 90) that complementarily binds to a nucleic acid sequence encoding DQ, the guide RNA (for example, SEQ ID NO: 123 or SEQ ID NO: 129) that complementarily binds to a nucleic acid sequence encoding DP, and the guide RNA (for example, SEQ ID NO: 186, SEQ ID NO: 188, or SEQ ID NO: 225) that complementarily binds to a nucleic acid sequence encoding DR. The modification of MHC II is performed together with the modification of MHC I, which may be performed by multiplex deletion using a multiple guide RNA (such as containing all of SEQ ID NO: 1, SEQ ID NO: 64, SEQ ID NO: 129, and SEQ ID NO: 188).

In an embodiment of the present invention, the transformed cell may be a therapeutic allogeneic cell. As used herein, the term "therapeutic allogeneic cell" refers to a non-autologous allogeneic cell to be injected into a subject for the purpose of suppressing progression of, treating, or alleviating symptoms of a disease, and examples thereof include, but are not limited to, immune cells and stem cells.

As used herein, the term "immune cell" refers to a cell involved in immune responses of the human body, and examples thereof include NK cells, T cells, B cells, dendritic cells, and macrophages.

In an embodiment of the present invention, the immune cell may be an NK cell or T cell.

As used herein, the term "stem cell" refers to a pluripotent cell capable of being differentiated into various cells. Examples of the stem cell may include human embryonic stem cells, bone marrow stem cells, mesenchymal stem cells, human nerve stem cells, oral mucosal cells, and the like. Specifically, the stem cell may be a mesenchymal stem cell.

In addition, in an aspect of the present invention, there is provided a pharmaceutical composition for treating cancer, an infectious disease, a degenerative disease, a hereditary disease, or an immune disease, comprising the transformed cell as an active ingredient.

In an embodiment of the present invention, the cancer may be any one selected from the group consisting of chronic lymphocytic leukemia (CLL), B-cell acute lymphocytic leukemia (B-ALL), acute lymphoblastic leukemia, acute myeloid leukemia, lymphoma, non-Hodgkin's lymphoma (NHL), multiple myeloma, blood cancer, gastric cancer, liver cancer, pancreatic cancer, colorectal cancer, lung cancer, breast cancer, ovarian cancer, skin cancer, melanoma, sarcoma, prostate cancer, esophageal cancer, hepatocellular carcinoma, astrocytoma, mesothelioma, head and neck cancer, and medulloblastoma.

In an embodiment of the present invention, the infectious disease may be any one selected from the group consisting of hepatitis B, hepatitis C, human papilloma virus (HPV) infection, cytomegalovirus infection, Epstein Barr virus (EBV) infection, viral respiratory disease, and influenza.

As used herein, the term "degenerative disease" refers to a pathological condition in which a tissue loses its original function due to irreversible quantitative loss of the tissue. Examples of the degenerative disease include, but are not limited to, brain neurological disease, ischemic disease, skin damage, bone disease, and degenerative arthritis.

As used herein, the term "hereditary disease" refers to a pathological condition that occurs due to a mutation that is harmful to a gene or chromosome. Examples of the hereditary disease include, but are not limited to, hemophilia, albinism, Fabry disease, Hunter syndrome, and glycogen storage disorder.

As used herein, the term "immune disease" refers to any pathological condition in which a tissue is damaged due to an excessive or undesired immune response. Accordingly, the term "immune disease" has the same meaning as "hyperactive immune disease", and the term "composition for preventing or treating an immune disease" has the same meaning as "immunosuppressant".

Examples of the immune disease include, but are not limited to, graft-versus-host disease, graft rejection, chronic inflammatory disease, inflammatory pain, neuropathic pain, chronic obstructive pulmonary disease (COPD), and autoimmune disease.

The term "autoimmune disease" refers to a pathological condition that occurs when immune cells fail to distinguish self from a foreign substance and thus attack the self. Examples of the autoimmune disease may include, but are not limited to, rheumatoid arthritis, systemic lupus erythematosis, Hashimoto's thyroiditis, Grave's disease, multiple sclerosis, scleroderma, myasthenia gravis, type I diabetes, allergic encephalomyelitis, glomerulonephritis, vitiligo, Behcet's disease, Crohn's disease, ankylosing spondylitis, thrombocytopenic purpura, pemphigus vulgaris, autoimmune hemolytic anemia, adrenoleukodystrophy (ALD), and systemic lupus erythematosus (SLE).

In an aspect of the present invention, there is provided a method for treating cancer, an infectious disease, a degenerative disease, a hereditary disease, or an immune disease, comprising administering the pharmaceutical composition to a subject.

In an embodiment of the present invention, the administration may be performed via any one route selected from the group consisting of intravenous, intramuscular, intradermal, subcutaneous, intraperitoneal, intraarteriolar, intraventricular, intralesional, intrathecal, topical, and combinations thereof.

In another aspect of the present invention, there is provided a use of a transformed cell for treating cancer, an infectious disease, a degenerative disease, a hereditary disease, or an immune disease, wherein expression of MHC I cell membrane receptor and MHC II cell membrane receptor is inhibited in the transformed cell, and the transformed cell expresses G-peptide bound to modified MHC I cell membrane receptor on the cell membrane surface.

In yet another aspect of the present invention, there is provided a kit for modifying a gene for MHC I cell membrane receptor and a gene for MHC II cell membrane receptor, the kit comprising the guide RNA or a nucleotide sequence encoding a guide RNA, and an RNA-guided endonuclease or a nucleotide sequence encoding the RNA-guided endonuclease.

### Mode for the Invention

Hereinafter, the present invention will be described in more detail by way of the following examples. However, the following examples are only for illustrating the present invention, and the scope of the present invention is not limited thereto.

### Example 1. Synthesis and selection of gRNA targeting HLA

### Example 1.1. Search and synthesis of gRNA sequence

In order to search for a gRNA sequence, the complete nucleotide sequences of genes provided by NCBI (https://www.ncbi.nlm.nih.gov/) were used. As design tools for gRNAs, web-based systems, CHOPCHOP (http://chopchop.cbu.uib.no/), E-CRISP (http://www.e-crisp.org/E-CRISP/designcrispr.html), CRISPR-ERA (http://crispr-era.stanford.edu/), RGEN Tools (http://www.rgenome.net/cas-designer/) were used. Among the designed gRNAs, about 60 gRNAs, which were most suitable for gene knock-out, were obtained per each desired target. Based on these sequences, gRNAs were synthesized using the GeneArt Precision gRNA Synthesis Kit (Thermo Fisher Scientific, A29377) according to the manufacturer's instructions.

That is, forward and reverse oligonucleotide primers, required to synthesize a DNA template encoding each of the gRNAs, were synthesized, and then PCR was performed with a PCR thermal cycler (FlexCycler2, Analytik Jena) using the synthesized primers and Tracr Fragment + T7 Primer Mix contained in the GeneArt Precision gRNA Synthesis Kit (Thermo Fisher Scientific, A29377). The following PCR parameters were used: pre-denaturation at 98°C for 10 seconds, followed by 32 cycles of denaturation and annealing under a condition of at 98°C for 5 seconds and at 55°C for 15 seconds, followed by final extension at 72°C for 1 minute. Using the obtained PCR product as a template, an in vitro transcription reaction was performed at 37°C for 4 hours; and then the resultant was purified to obtain a gRNA.

The obtained gRNAs are shown in Tables 1 to 4 below. Specifically, the gRNA sequences for HLA-ABC (B2M) are shown in Table 1; the gRNA sequences for HLA-DQ are shown in Table 2; the gRNA sequences for HLA-DP are shown in Table 3 below; and the gRNA sequences for HLA-DR are shown in Table 4 below.

**[Table 1]**

| HLA-ABC | gRNA sequence | SEQ ID NO |
|---|---|---|
| B2M-01 | GAGUAGCGCGAGCACAGCUA | SEQ ID NO: 1 |
| B2M-03 | CUCGCGCUACUCUCUCUUUC | SEQ ID NO: 2 |
| B2M-04 | GCAUACUCAUCUUUUUCAGU | SEQ ID NO: 3 |
| B2M-05 | GCUACUCUCUCUUUCUGGCC | SEQ ID NO: 4 |
| B2M-06 | GGCAUACUCAUCUUUUUCAG | SEQ ID NO: 5 |
| B2M-07 | GGCCACGGAGCGAGACAUCU | SEQ ID NO: 6 |
| B2M-08 | GGCCGAGAUGUCUCGCUCCG | SEQ ID NO: 7 |
| B2M-09 | UCACGUCAUCCAGCAGAGAA | SEQ ID NO: 8 |
| B2M-10 | ACAAAGUCACAUGGUUCACA | SEQ ID NO: 9 |
| B2M-11 | AGUCACAUGGUUCACACGGC | SEQ ID NO: 10 |
| B2M-12 | AAGUCAACUUCAAUGUCGGA | SEQ ID NO: 11 |
| B2M-13 | CAUACUCAUCUUUUUCAGUG | SEQ ID NO: 12 |
| B2M-14 | UCCUGAAUUGCUAUGUGUCU | SEQ ID NO: 13 |
| B2M-15 | CGUGAGUAAACCUGAAUCUU | SEQ ID NO: 14 |
| B2M-16 | UUGGAGUACCUGAGGAAUAU | SEQ ID NO: 15 |
| B2M-17 | AGGGUAGGAGAGACUCACGC | SEQ ID NO: 16 |
| B2M-18 | ACAGCCCAAGAUAGUUAAGU | SEQ ID NO:17 |
| B2M-19 | AUACUCAUCUUUUUCAGUGG | SEQ ID NO: 18 |
| B2M-20 | UGGAGUACCUGAGGAAUAUC | SEQ ID NO: 19 |
| B2M-21 | AAGAAAAGGAAACUGAAAAC | SEQ ID NO: 20 |
| B2M-22 | AAGAAGGCAUGCACUAGACU | SEQ ID NO: 21 |
| B2M-23 | ACAUGUAAGCAGCAUCAUGG | SEQ ID NO: 22 |
| B2M-24 | ACCCAGACACAUAGCAAUUC | SEQ ID NO: 23 |
| B2M-25 | ACUUGUCUUUCAGCAAGGAC | SEQ ID NO: 24 |
| B2M-26 | CAAGCCAGCGACGCAGUGCC | SEQ ID NO: 25 |
| B2M-27 | CACAGCCCAAGAUAGUUAAG | SEQ ID NO: 26 |
| B2M-29 | CAUCACGAGACUCUAAGAAA | SEQ ID NO: 27 |
| B2M-30 | CGCAGUGCCAGGUUAGAGAG | SEQ ID NO: 28 |
| B2M-31 | CUAACCUGGCACUGCGUCGC | SEQ ID NO: 29 |
| B2M-32 | GAAAGUCCCUCUCUCUAACC | SEQ ID NO: 30 |
| B2M-33 | GAGACAUGUAAGCAGCAUCA | SEQ ID NO: 31 |
| B2M-34 | GAGUCUCGUGAUGUUUAAGA | SEQ ID NO: 32 |
| B2M-35 | GCAGUGCCAGGUUAGAGAGA | SEQ ID NO: 33 |
| B2M-36 | UAAGAAGGCAUGCACUAGAC | SEQ ID NO: 34 |
| B2M-37 | UCGAUCUAUGAAAAAGACAG | SEQ ID NO: 35 |
| B2M-39 | UUCAGACUUGUCUUUCAGCA | SEQ ID NO: 36 |
| B2M-40 | UUCCUGAAUUGCUAUGUGUC | SEQ ID NO: 37 |
| B2M-41 | UAAGAAAAGGAAACUGAAAA | SEQ ID NO: 38 |
| B2M-42 | CUGGCACUGCGUCGCUGGCU | SEQ ID NO: 39 |
| B2M-43 | UGCGUCGCUGGCUUGGAGAC | SEQ ID NO: 40 |
| B2M-44 | GCUGGCUUGGAGACAGGUGA | SEQ ID NO: 41 |
| B2M-45 | AGACAGGUGACGGUCCCUGC | SEQ ID NO: 42 |
| B2M-46 | CAAUCAGGACAAGGCCCGCA | SEQ ID NO: 43 |
| B2M-47 | CCUGCGGGCCUUGUCCUGAU | SEQ ID NO: 44 |
| B2M-48 | CCAAUCAGGACAAGGCCCGC | SEQ ID NO: 45 |
| B2M-49 | CGGGCCUUGUCCUGAUUGGC | SEQ ID NO: 46 |
| B2M-50 | GGGCCUUGUCCUGAUUGGCU | SEQ ID NO: 47 |
| B2M-51 | GUGCCCAGCCAAUCAGGACA | SEQ ID NO: 48 |
| B2M-52 | AAACGCGUGCCCAGCCAAUC | SEQ ID NO: 49 |
| B2M-53 | GGGCACGCGUUUAAUAUAAG | SEQ ID NO: 50 |
| B2M-54 | CACGCGUUUAAUAUAAGUGG | SEQ ID NO: 51 |
| B2M-55 | UAUAAGUGGAGGCGUCGCGC | SEQ ID NO: 52 |
| B2M-56 | AAGUGGAGGCGUCGCGCUGG | SEQ ID NO: 53 |
| B2M-57 | AGUGGAGGCGUCGCGCUGGC | SEQ ID NO: 54 |
| B2M-58 | UUCCUGAAGCUGACAGCAUU | SEQ ID NO: 55 |
| B2M-59 | UCCUGAAGCUGACAGCAUUC | SEQ ID NO: 56 |
| B2M-60 | UGGGCUGUGACAAAGUCACA | SEQ ID NO: 57 |
| B2M-61 | ACUCUCUCUUUCUGGCCUGG | SEQ ID NO: 58 |

**[Table 2]**

| HLA-DQ | gRNA sequence | SEQ ID NO |
|---|---|---|
| DQA-08 | UUAGGAUCAUCCUCUUCCCA | SEQ ID NO: 59 |
| DQA-09 | AACUCUACCGCUGCUACCAA | SEQ ID NO: 60 |
| DQA-10 | ACAAUGUCUUCACCUCCACA | SEQ ID NO: 61 |
| DQA-11 | ACCACCGUGAUGAGCCCCUG | SEQ ID NO: 62 |
| DQA-12 | ACCCAGUGUCACGGGAGACU | SEQ ID NO: 63 |
| DQA-14 | ACCUCCACAGGGGCUCAUCA | SEQ ID NO: 64 |
| DQA-15 | CAAUGUCUUCACCUCCACAG | SEQ ID NO: 65 |
| DQA-16 | CACAAUGUCUUCACCUCCAC | SEQ ID NO: 66 |
| DQA-17 | CAGUACACCCAUGAAUUUGA | SEQ ID NO: 67 |
| DQA-18 | CUCUGUGAGCUCUGACAUAG | SEQ ID NO: 68 |
| DQA-19 | CUGUGGAGGUGAAGACAUUG | SEQ ID NO: 69 |
| DQA-20 | GGCUGGAAUCUCAGGCUCUG | SEQ ID NO: 70 |
| DQA-21 | GUUGGGCUGACCCAGUGUCA | SEQ ID NO: 71 |
| DQA-22 | UCAUGGGUGUACUGGCCAGA | SEQ ID NO: 72 |
| DQA-23 | UCCAAGUCUCCCGUGACACU | SEQ ID NO: 73 |
| DQA-24 | UCCACAGGGGCUCAUCACGG | SEQ ID NO: 74 |
| DQA-25 | UGUGGAGGUGAAGACAUUGU | SEQ ID NO: 75 |
| DQA-26 | UUCCAAGUCUCCCGUGACAC | SEQ ID NO: 76 |
| DQA-27 | UUGGGCUGACCCAGUGUCAC | SEQ ID NO: 77 |
| DQA-28 | AACAUCACAUGGCUGAGCAA | SEQ ID NO: 78 |
| DQA-29 | ACAUCACAUGGCUGAGCAAU | SEQ ID NO: 79 |
| DQA-30 | AGCCAUGUGAUGUUGACCAC | SEQ ID NO: 80 |
| DQA-31 | AGGAAUGAUCACUCUUGGAG | SEQ ID NO: 81 |
| DQA-32 | AUCACUCUUGGAGAGGAAGC | SEQ ID NO: 82 |
| DQA-33 | AUGACUGCAAGGUGGAGCAC | SEQ ID NO: 83 |
| DQA-34 | CAAGGUGGAGCACUGGGGCC | SEQ ID NO: 84 |
| DQA-35 | CAUCAAAUUCAUGGGUGUAC | SEQ ID NO: 85 |
| DQA-36 | CAUGUGAUGUUGACCACAGG | SEQ ID NO: 86 |
| DQA-37 | CCUCACCACAGAGGUUCCUG | SEQ ID NO: 87 |
| DQA-38 | CUCAUCUCCAUCAAAUUCAU | SEQ ID NO: 88 |
| DQA-39 | CUCCUGUGGUCAACAUCACA | SEQ ID NO: 89 |
| DQA-40 | GAAGAAGGAAUGAUCACUCU | SEQ ID NO: 90 |
| DQA-41 | GACUGCAAGGUGGAGCACUG | SEQ ID NO: 91 |
| DQA-42 | GAGGUAACUGAUCUUGAAGA | SEQ ID NO: 92 |
| DQA-43 | GGACAACAUCUUUCCUCCUG | SEQ ID NO: 93 |
| DQA-44 | GUGCUGUUUCCUCACCACAG | SEQ ID NO: 94 |
| DQA-45 | UCUUCUGAAACACUGGGGUA | SEQ ID NO: 95 |
| DQA-47 | UUCAUGGGUGUACUGGCCAG | SEQ ID NO: 96 |
| DQA-48 | AGAGACUGUGGUCUGCGCCC | SEQ ID NO: 97 |
| DQA-49 | GACAUAGGGGCUGGAAUCUC | SEQ ID NO: 98 |
| DQA-50 | GAGACUGUGGUCUGCGCCCU | SEQ ID NO: 99 |
| DQA-51 | GGCCUCGUGGGCAUUGUGGU | SEQ ID NO: 100 |
| DQA-52 | GGGCCUCGUGGGCAUUGUGG | SEQ ID NO: 101 |
| DQA-53 | GUCAGAGCUCACAGAGACUG | SEQ ID NO: 102 |
| DQA-54 | GUCUCUGUGAGCUCUGACAU | SEQ ID NO: 103 |
| DQA-55 | GUGAGCUCUGACAUAGGGGC | SEQ ID NO: 104 |
| DQA-56 | GUUGGUGCUUCCAGACACCA | SEQ ID NO: 105 |
| DQA-57 | UCUCUGUGAGCUCUGACAUA | SEQ ID NO: 106 |
| DQA-58 | UGACUGCAAGGUGGAGCACU | SEQ ID NO: 107 |
| DQA-59 | UGCCCACCACAAUGCCCACG | SEQ ID NO: 108 |
| DQA-60 | UGGAAGCACCAACUGAACGC | SEQ ID NO: 109 |
| DQA-61 | UGUGGGCCUCGUGGGCAUUG | SEQ ID NO: 110 |
| DQA-62 | UUACCCCAGUGUUUCAGAAG | SEQ ID NO: 111 |
| BQA-65 | UUGGAAAACACUGUGACCUC | SEQ ID NO: 112 |
| DQA-64 | UUGGUGCUUCCAGACACCAA | SEQ ID NO: 113 |
| DQA-65 | AAACAAAGCUCUGCUGCUGG | SEQ ID NO: 114 |
| DQA-66 | AAAUCUCAUCAGCAGAAGGG | SEQ ID NO: 115 |
| DQA-67 | CUAAACAAAGCUCUGCUGCU | SEQ ID NO: 116 |

**[Table 3]**

| HLA-DP | gRNA sequence | SEQ ID NO |
|---|---|---|
| DPA-01 | UCUAUGCGUCUGUACAAACG | SEQ ID NO: 117 |
| DPA-02 | GUACAGACGCAUAGACCAAC | SEQ ID NO: 118 |
| DPA-03 | UACAGACGCAUAGACCAACA | SEQ ID NO: 119 |
| DPA-04 | GAAGGAGACCGUCUGGCAUC | SEQ ID NO: 120 |
| DPA-05 | GGAGACCGUCUGGCAUCUGG | SEQ ID NO: 121 |
| DPA-06 | GUCUGGCAUCUGGAGGAGUU | SEQ ID NO: 122 |
| DPA-07 | GUGGUUGGAACGCUGGAUCA | SEQ ID NO: 123 |
| DPA-08 | GUCUUCAGGGCGCAUGUUGU | SEQ ID NO: 124 |
| DPA-09 | UGUCUUCAGGGCGCAUGUUG | SEQ ID NO: 125 |
| DPA-10 | UCUUCAGGGCGCAUGUUGUG | SEQ ID NO: 126 |
| DPA-11 | GUUGCAUACCCCAGUGCUUG | SEQ ID NO: 127 |
| DPA-12 | GACCUUUGUGCCCUCAGCAG | SEQ ID NO: 128 |
| DPA-13 | GAGACUCAGCAGGAAAGCCA | SEQ ID NO: 129 |
| DPA-14 | GAGCCUCAAAGGAAAAGGCU | SEQ ID NO: 130 |
| DPA-15 | GAUCUUGAGAGCCCUCUCCU | SEQ ID NO: 131 |
| DPA-16 | GCCAUCAAGGGUGAGUGCUC | SEQ ID NO: 132 |
| DPA-17 | GCCAUGACCCCCGGGCCCAG | SEQ ID NO: 133 |
| DPA-18 | GCCCAGCUCCACAGGCUCCU | SEQ ID NO: 134 |
| DPA-19 | GCCCUGAGCCUCAAAGGAAA | SEQ ID NO: 135 |
| DPA-20 | GCCUUUUCCUUUGAGGCUCA | SEQ ID NO: 136 |
| DPA-21 | GCGUUCUGGCCAUGACCCCC | SEQ ID NO: 137 |
| DPA-22 | GCUUUCCUGCUGAGUCUCCG | SEQ ID NO: 138 |
| DPA-23 | GGAAACACGGUCACCUCAGG | SEQ ID NO: 139 |
| DPA-24 | GGACUUCUAUGACUGCAGGG | SEQ ID NO: 140 |
| DPA-25 | GGAGACUGUGCUCUGUGCCC | SEQ ID NO: 141 |
| DPA-16 | GGCCAUGACCCCCGGGCCCA | SEQ ID NO: 142 |
| DPA-27 | GGCCUAGUCGGCAUCAUCGU | SEQ ID NO: 143 |
| DPA-29 | GGGAAACACGGUCACCUCAG | SEQ ID NO: 144 |
| DPA-30 | GGGCCUAGUCGGCAUCAUCG | SEQ ID NO: 145 |
| DPA-31 | GUCAUAGAAGUCCUCUGCUG | SEQ ID NO: 146 |
| DPA-32 | GUCCUCUGCUGAGGGCACAA | SEQ ID NO: 147 |
| DPA-33 | GUGGAAGCUGUAAUCUGUUC | SEQ ID NO: 148 |
| DPA-34 | GUGGGAAGAACUUGUCAAUG | SEQ ID NO: 149 |
| DPA-35 | GUUGGUGGCCUGAGUGUGGU | SEQ ID NO: 150 |
| DPA-36 | GUUGUCUCAGGCAUCUGGAU | SEQ ID NO: 151 |
| DPA-37 | GCUGAGUCUCCGAGGAGCUG | SEQ ID NO: 152 |
| DPA-38 | UCUCUACUGUCUUUAUGCAG | SEQ ID NO: 153 |
| DPA-39 | UAUGGAACAUUCUGUCUUCA | SEQ ID NO: 154 |
| DPA-40 | UCAAGAUCACAGCUCUGAUA | SEQ ID NO: 155 |
| DPA-41 | UCAAACAUAAACUCCCCUGU | SEQ ID NO: 156 |
| DPA-42 | UACCGUUGGUGGCCUGAGUG | SEQ ID NO: 157 |
| DPA-43 | UCCUGAGCACUCACCCUUGA | SEQ ID NO: 158 |
| DPA-44 | UGAGGUGACCGUGUUUCCCA | SEQ ID NO: 159 |
| DPA-45 | UGCGUUCUGGCCAUGACCCC | SEQ ID NO: 160 |
| DPA-46 | UUUCCUUUGAGGCUCAGGGC | SEQ ID NO: 161 |
| DPA-47 | UGCCGACUAGGCCCAGCACC | SEQ ID NO: 162 |
| DPA-48 | UCAGCAGGAAAGCCAAGGAG | SEQ ID NO: 163 |
| DPA-49 | UGAAGAUGAGAUGUUCUAUG | SEQ ID NO: 164 |
| DPA-50 | UGCUGAGUCUCCGAGGAGCU | SEQ ID NO: 165 |
| DPA-51 | UGAGAUGUUCUAUGUGGAUC | SEQ ID NO: 166 |
| DPA-52 | UGGGAAACACGGUCACCUCA | SEQ ID NO: 167 |
| DPA-53 | UGGAAGCUGUAAUCUGUUCU | SEQ ID NO: 168 |
| DPA-54 | UGGACAAGAAGGAGACCGUC | SEQ ID NO: 169 |
| DPA-55 | UGCCCACGAUGAUGCCGACU | SEQ ID NO: 170 |
| DPA-56 | UGGCCAAGCCUUUUCCUUUG | SEQ ID NO: 171 |
| DPA-57 | GUGGCUGUGCAACGGGGAGC | SEQ ID NO: 172 |
| DPA-58 | UCCCCUGGGCCCGGGGGUCA | SEQ ID NO: 173 |
| DPA-59 | UCACCUCAGGGGGAUCUGGA | SEQ ID NO: 174 |
| DPA-60 | UCUCCUUCCAGAUCCCCCUG | SEQ ID NO: 175 |

**[Table 4]**

| HLA-DR | gRNA sequence | SEQ ID NO |
|---|---|---|
| DRA-08 | AAGAAGAAAAUGGCCAUAAG | SEQ ID NO: 176 |
| DRA-09 | AAUCAUGGGCUAUCAAAGGU | SEQ ID NO: 177 |
| DRA-10 | AGCUGUGCUGAUGAGCGCUC | SEQ ID NO: 178 |
| DRA-11 | AUAAGUGGAGUCCCUGUGCU | SEQ ID NO: 179 |
| DRA-12 | ACUUAUGGCCAUUUUCUUCU | SEQ ID NO: 180 |
| DRA-13 | AUGAUGAAAAAUCCUAGCAC | SEQ ID NO: 181 |
| DRA-14 | CAGAGCGCCCAAGAAGAAAA | SEQ ID NO: 182 |
| DRA-15 | CAGGAAUCAUGGGCUAUCAA | SEQ ID NO: 183 |
| DRA-16 | CUUAUGGCCAUUUUCUUCUU | SEQ ID NO: 184 |
| DRA-17 | GACUGUCUCUGACACUCCUG | SEQ ID NO: 185 |
| DRA-18 | GAGCCUCUUCUCAAGCACUG | SEQ ID NO: 186 |
| DRA-19 | GAUAGUGGAACUUGCGGAAA | SEQ ID NO: 187 |
| DRA-20 | GAUGAGCGCUCAGGAAUCAU | SEQ ID NO: 188 |
| DRA-21 | GCUAUCAAAGGUAGGUGCUG | SEQ ID NO: 189 |
| DRA-22 | GUUACCUCUGGAGGUACUGG | SEQ ID NO: 190 |
| DRA-23 | UAGCACAGGGACUCCACUUA | SEQ ID NO: 191 |
| DRA-24 | UGAUGAAAAAUCCUAGCACA | SEQ ID NO: 192 |
| DRA-25 | UGAUGAGCGCUCAGGAAUCA | SEQ ID NO: 193 |
| DRA-27 | UUUGCCAGCUUUGAGGCUCA | SEQ ID NO: 194 |
| DRA-28 | AACUAUACUCCGAUCACCAA | SEQ ID NO: 195 |
| DRA-29 | AGAAGAACAUGUGAUCAUCC | SEQ ID NO: 196 |
| DRA-30 | AGCAGAGAGGGAGGUACCAU | SEQ ID NO: 197 |
| DRA-31 | AGCGCUUUGUCAUGAUUUCC | SEQ ID NO: 198 |
| DRA-32 | AGCUGUGGACAAAGCCAACC | SEQ ID NO: 199 |
| DRA-33 | AGGGAGGUACCAUUGGUGAU | SEQ ID NO: 200 |
| DRA-34 | AUAAACUCGCCUGAUUGGUC | SEQ ID NO: 201 |
| DRA-35 | AUUGGUGAUCGGAGUAUAGU | SEQ ID NO: 202 |
| DRA-36 | CCAUGUGGAUAUGGCAAAGA | SEQ ID NO: 203 |
| DRA-37 | CUUUGAGGCUCAAGGUGCAU | SEQ ID NO: 204 |
| DRA-38 | CUUUGUCAUGAUUUCCAGGU | SEQ ID NO: 205 |
| DRA-39 | GGAUAUGGCAAAGAAGGAGA | SEQ ID NO: 206 |
| DRA-40 | UAUCUGAAUCCUGACCAAUC | SEQ ID NO: 207 |
| DRA-41 | UGAGAUUUUCCAUGUGGAUA | SEQ ID NO: 208 |
| DRA-42 | UGAUCACAUGUUCUUCUGAA | SEQ ID NO: 209 |
| DRA-43 | UGCACCUUGAGCCUCAAAGC | SEQ ID NO: 210 |
| DRA-44 | UGCAUUGGCCAACAUAGCUG | SEQ ID NO: 211 |
| DRA-45 | UGGACGAUUUGCCAGCUUUG | SEQ ID NO: 212 |
| DRA-46 | UGGCAAAGAAGGAGACGGUC | SEQ ID NO: 213 |
| DRA-47 | UGGUGAUGAGAUUUUCCAUG | SEQ ID NO: 214 |
| DRA-48 | AAUGUCACGUGGCUUCGAAA | SEQ ID NO: 215 |
| DRA-49 | AGACAAGUUCACCCCACCAG | SEQ ID NO: 216 |
| DRA-50 | CAAUCCCUUGAUGAUGAAGA | SEQ ID NO: 217 |
| DRA-51 | GAACGCAGGGGGCCUCUGUA | SEQ ID NO: 218 |
| DRA-52 | CUGAGGACGUUUACGACUGC | SEQ ID NO: 219 |
| DRA-53 | GCGGAAAAGGUGGUCUUCCC | SEQ ID NO: 220 |
| DRA-54 | GGACGUUUACGACUGCAGGG | SEQ ID NO: 221 |
| DRA-55 | GUCGUAAACGUCCUCAGUUG | SEQ ID NO: 222 |
| DRA-56 | GUGAGCACAGUUACCUCUGG | SEQ ID NO: 223 |
| DRA-57 | GUGUCCCCCAGUACCUCCAG | SEQ ID NO: 224 |
| DRA-58 | UGAGGACGUUUACGACUGCA | SEQ ID NO: 225 |
| DRA-59 | AAUGGAAAACCUGUCACCAC | SEQ ID NO: 226 |
| DRA-60 | AGUGGAACUUGCGGAAAAGG | SEQ ID NO: 227 |
| DRA-61 | AUGAAACAGAUGAGGACGUU | SEQ ID NO: 228 |
| DRA-62 | CAGAGACAGUCUUCCUGCCC | SEQ ID NO: 229 |
| DRA-63 | CGUGACAUUGACCACUGGUG | SEQ ID NO: 230 |
| DRA-64 | UAUGAAACAGAUGAGGACGU | SEQ ID NO: 231 |
| DRA-65 | UCUGACACUCCUGUGGUGAC | SEQ ID NO: 232 |
| DRA-66 | AAACGUCCUCAGUUGAGGGC | SEQ ID NO: 233 |
| DRA-67 | UCGUAAACGUCCUCAGUUGA | SEQ ID NO: 234 |

### Example 1.2. Selection of gRNA through transfection into Raji cell line

7.5 µg of the obtained gRNA was incubated at 65°C for 10 minutes to form a single strand. Then, 7.5 µg of Cas9 protein (Toolgen, TGEN_CP3 or Clontech, M0646T) was added thereto and incubation was performed at 25°C for 10 minutes to prepare a Cas9-gRNA complex (RNP complex). The RNP complex was transfected into Raji cell line having 4 × 10⁵ cells with 4D-Nucleofector™ X Unit (Lonza, AAF-1002X) using SG Cell Line 4D-Nucleofector® X Kit S (Lonza, V4XC-3032). The transfected cells were incubated for 7 days, and then the expression level of HLA on the cell surface and the presence of a mutation in genomic DNA were identified.

### Example 1.3. Identification of expression level of HLA using flow cytometer

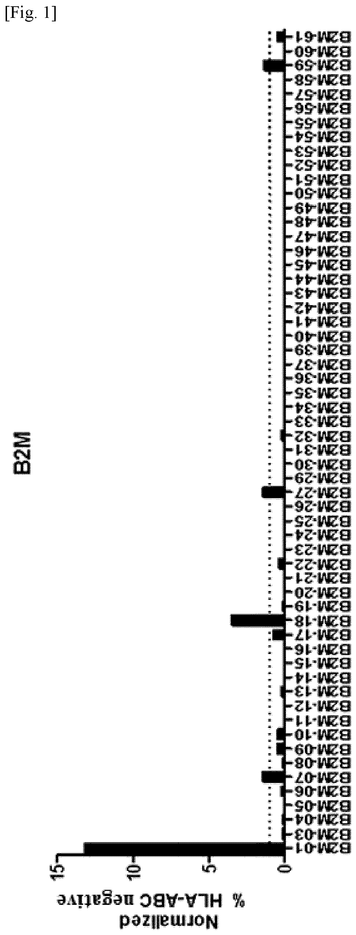

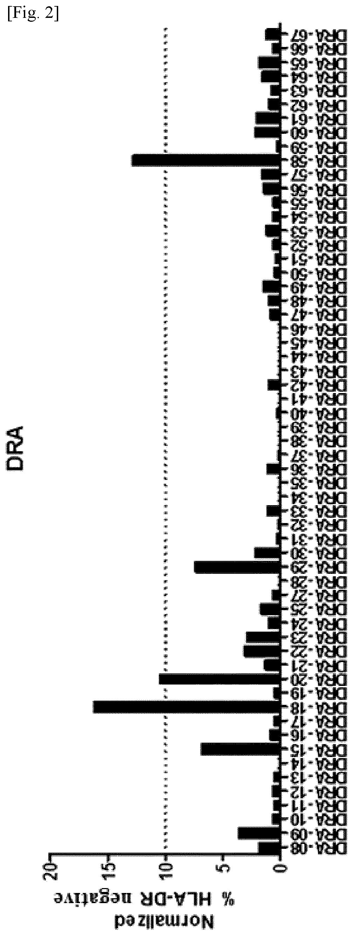

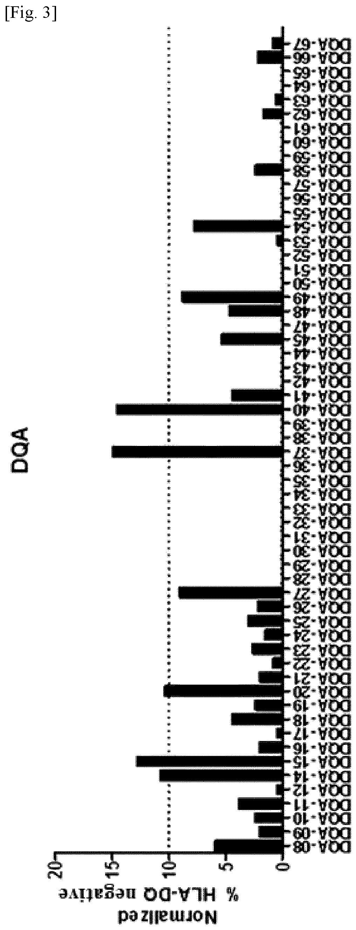

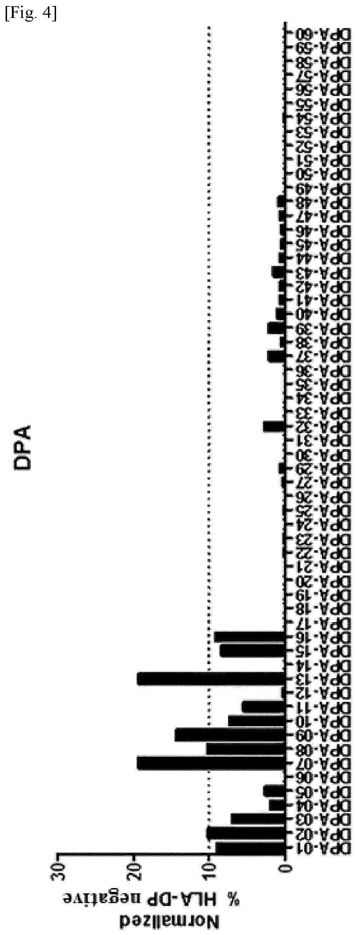

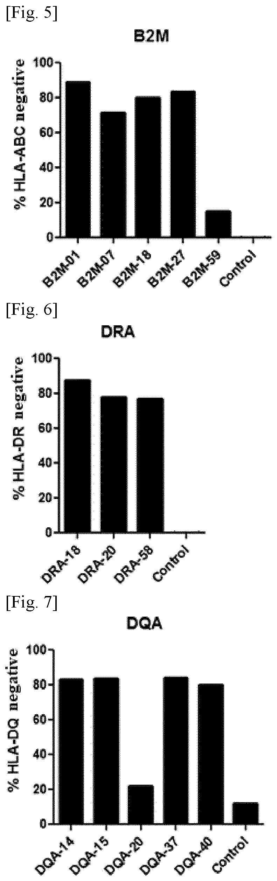

2 × 10⁵ cells of each of the RNP complex-transfected Raji cell line and control group Raji cell line were suspended in 100 µL of FACS buffer (1% FBS/sheath buffer) and prepared in a 5-mL tube. The cells were subjected to antibody treatment. Then, light was blocked for 30 minutes and incubation was performed at 4°C. As the antibodies, PE anti-HLA-ABC (Miltenyi Biotec, 130-101-448), PE anti-HLA-DR (Biolegend, 361605), PE anti-HLA-DQ (Biolegend, 318106), and PE anti-HLA-DP (Leinco Technologies, H130) were used. Thereafter, 3 mL of FACS buffer was added thereto and centrifugation was performed at 2,000 rpm for 3 minutes at 4°C. Then, the supernatant was removed to obtain a sample, and the sample was analyzed by LSR Fortessa. A total of 60 gRNAs were tested three times, each time using 20 gRNAs. The results for a value (normalized% HLA negative) calculated by subtracting the '% HLA negative' value of the control group from the '% HLA negative' value of each gRNA are illustrated in Figs. 1 to 4. In addition, the results obtained by performing a re-experiment, at once, on the gRNAs having the value of 10 or higher (however, on the gRNAs having the value of 1 or higher in case of B2M-targeted gRNAs) are illustrated in Figs. 5 to 8.

From the results in Figs. 5 to 8, a total of 13 gRNAs capable of efficiently decreasing expression of each HLA were selected, of which 2 to 4 gRNAs were selected for respective targets (HLA-ABC, HLA-DQ, HLA-DP, and HLA-DR). Specifically, B2M-01, B2M-07, B2M-18, and B2M-27 gRNAs were selected for HLA-ABC; DQA-14, DQA-15, DQA-37, and DQA-40 were selected for HLA-DQ; DPA-07 and DPA-13 were selected for HLA-DP; and DRA-18, DRA-20, and DRA-58 were selected for HLA-DR.

### Example 1.4. Identification of mutation in genomic DNA of target gene

In order to identify whether the HLA-targeted gRNAs selected using flow cytometry cause a mutation in genomic DNA, the genomic DNA was analyzed using the Guide-it Mutation Detection Kit (Clontech, 631443) according to the manufacturer's instructions.

That is, 5 × 10⁵ cells of each of the RNP complex-transfected Raji cell line and the control group Raji cell line were centrifuged at 1,200 rpm for 5 minutes, and then the supernatant was removed. Then, 90 µL of extraction buffer 1 contained in the Guide-it Mutation Detection Kit (Clontech, 631443) was added thereto, and incubation was performed at 95°C for 10 minutes. Then, 10 µL of extraction buffer 2 contained in the Guide-it Mutation Detection Kit (Clontech, 631443) was added thereto, and the DNA lysate obtained by pipetting was diluted in a ratio of 1:8 in pure water for PCR. PCR was performed with a PCR thermal cycler (FlexCycler2, Analytik Jena) using the diluted DNA lysate, and the selected gRNA and the analytical PCR primers for target genomic DNA as shown in Table 5 below.

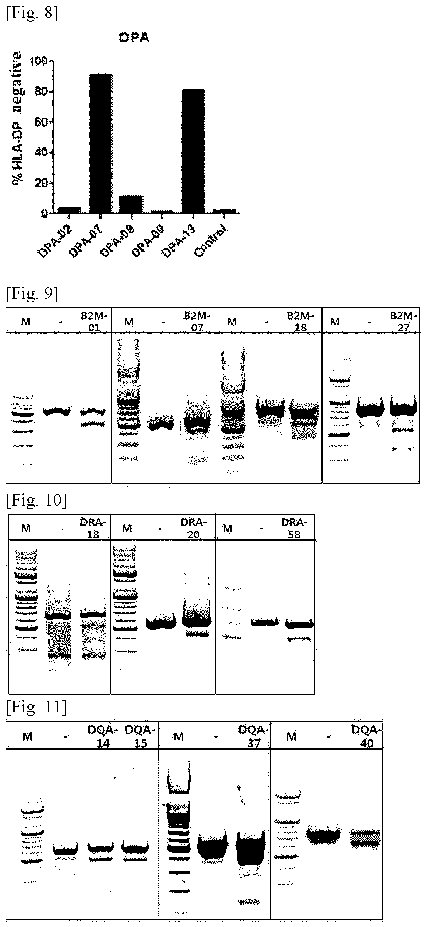

The following PCR parameters were used to produce the PCT product: pre-denaturation at 98°C for 2 minutes, followed by 35 cycles of denaturation and annealing under a condition of at 98°C for 10 seconds, at 60°C for 15 seconds, and at 68°C for 1 minute, followed by extension at 68°C for 5 minutes. To denature and rehybridize the obtained PCR product, 5 µL of pure water for PCR was added to 10 µL of the PCR product. Subsequently, incubation was performed at 95°C for 5 minutes, and then the temperature was changed under a condition where the temperature decreased by 2°C per second from 95°C to 85°C and decreased by 0.1°C per second from 85°C to 25°C. Finally, 1 µL of Guide-it Resolvase was added thereto and incubation was performed at 37°C for 30 minutes. Then, electrophoresis was performed on 1.5% agarose gel. The results are illustrated in Figs. 9 to 12.

Guide-it resolvase-cleaved DNA fragments in the PCT product of the HLA-targeted gRNA-transfected cells were identified in the electrophoresis results. From these results, it was found that the 13 selected HLA-targeted gRNAs induced a mutation on their target genomic DNA.

**[Table 5]**

| **gRNA name** | **Estimated size of cleared DNA(bp)** | | **PCR primer sequence** |
|---|---|---|---|
| **B2M-1** | **173·303** | **B2M E1-1F** | **CTGGCTTGGIGICISSTSIC** (SEQ ID NO: 242) |
| | | **B2M E1-1B** | **GACGCTTATCGACGCCTAA** (SEQ ID NO: 243) |
| **B2M-7** | **122·359** | **B2M E1-1F** | **CTGGCTTGGAGACAGGTGAC** (SEQ ID NO: 242) |
| | | **B2M E1-1B** | **GACGCTTATCGACGCCCTAA** (SEQ ID NO: 243) |
| **B2M-18** | **326·474** | **B2M E2-2F** | **CCCAAGTGAAATACCCTGGCA** (SEQ ID NO: 244) |
| | | **B2M E2-2R** | **AGCCTTCCTACTAGCCTCA** (SEQ ID NO: 245) |
| **B2M-27** | **325·475** | **B2M E2-2F** | **CCCAAGTSAAATACCTGGCA** (SEQ ID NO: 244) |
| | | **B2M E2-2R** | **AGCCCTTCCTACTAGCCTCA** (SEQ ID NO: 245) |
| **DRA-18** | **475·131** | **DRA E3-1F** | **AATTTCTTGGGGAGGGGGTG** (SEQ ID NO: 246) |
| | | **DEA E3-1R** | **AGCTGGATAGTAGGAGAAGACAGT** (SEQ ID NO: 247) |
| **DRA-20** | **382·141** | **DRA E1-3F** | **GGGTTAAAGAGTCTGTCCGTGA** (SEQ ID NO: 248) |
| | | **DEA E1-3R** | **TGTCGAGACCACATAATACCTGT** (SEQ ID NO: 249) |
| **DRA-58** | **176·430** | **DRA E3-1F** | **AATTTCTTGGGGAGGGGTG** (SEQ ID NO: 246) |
| | | **DRA E3-1R** | **AGCTGGATAGTAGGAGAAGACAGT** (SEQ ID NO: 247) |
| **DOA-14** | **163·433** | **DOA E1-1F** | **ACCTGACTTGGCAGGGTTTG** (SEQ ID NO: 250) |
| | | **DOA E1-1R** | **ACCTGACTTGGCAGGGTTTG** (SEQ ID NO:251) |
| **DOA-15** | **173·423** | **DOA E1-1F** | **ACCTGACTTGGCAGGGTTTG** (SEQ ID NO: 250) |
| | | **DOA E1-1E** | **CCCAAGATCTACCACCGAGA** (SEQ ID NO: 251) |
| **DOA-37** | **146·383** | **DOA E3-2F** | **TGCTCCCAAGCAGAAGGTAA** (SEQ ID NO: 252) |
| | | **DOA E3-2R** | **AACCCATGAAGTGGAAAACAAG** (SEQ ID NO: 253) |
| **DOA-40** | **127·543** | **DOA E3-1F** | **TCCCTCCATACCAGGGTTCA** (SEQ ID NO:254) |
| | | **DOA E3-1R** | **AACTCATCCTTACCCCAGTGT** (SEQ ID NO: 255) |
| **DPA-7** | **206·401** | **DPA 5F** | **TGTGTCAACTTATGCCGCT** (SEQ ID NO: 256) |
| | | **DPA 5R** | **TTGGGAAACACGGTCACCTC** (SEQ ID NO: 257) |
| **DPA-13** | **178·322** | **DPA E1-2F** | **TGTGAACTGGAGCTCTCTTGA** (SEQ ID NO: 25B) |
| | | **DPA E1-2R** | **TATGAGGGCCAGAGGGAACAT** (SEQ ID NO: 259) |

As such, the gRNAs capable of efficiently decreasing expression of respective HLAs through transfection in Raji cells were selected. In Examples 2 and 3, the selected gRNAs were used to prepare transformed NK cells, and then efficacy thereof was identified.

### Example 2. Preparation and identification of HLA-I-deleted cells

### Example 2.1. Deletion of HLA-I in NK-92MI cell line

37.5 µg of B2M-01 gRNA was incubated at 65°C for 10 minutes to form a single strand. Then, 37.5 µg of Cas9 protein (Toolgen, TGEN_CP3) was added thereto and incubation was performed at 25°C for 10 minutes to prepare a Cas9-gRNA complex (RNP complex). The RNP complex was transfected into NK-92MI cell line having 2 × 10⁶ cells with Nucleofector™ 2b (Lonza, AAB-1001) using Cell Line nucleofector Kit R (Lonza, VCA-1001). The transfected cells were incubated for 3 days, and then cell separation was performed using a cell separator.

### Example 2.2. Separation of HLA I negative cells

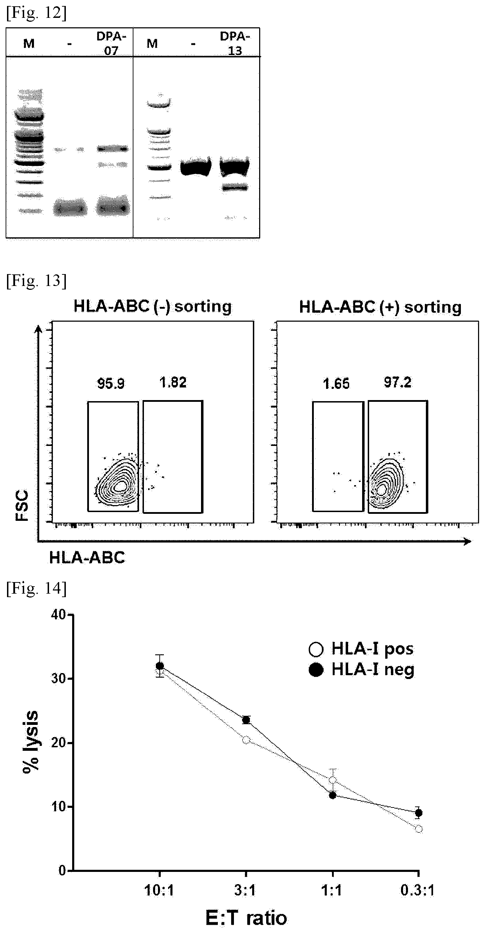

The B2M-01 RNP complex-transfected NK-92MI cell line was transferred to a 5-mL tube, and then treated with PE anti-HLA-ABC (Miltenyi Biotec, 130-101-448) and 7-AAD (Beckman Coulter, Inc., A07704). Then, light was blocked for 30 minutes and incubation was performed at 4°C. The stained cells were filtered using a filter top FACS tube (Falcon, 352235), and then HLA-I positive cells and HLA-I negative cells were separated using FACS Aria II (BD). The results are illustrated in Fig. 13. It was identified that the HLA-I negative cells have a purity of 95.9% and the HLA-I positive cells have a purity of 97.2%.

### Example 2.3. Evaluation of cell-killing capacity of HLA-I-deleted NK-92MI cell line

Using the HLA-I positive cells and the HLA-I negative cells, each of which had been incubated for 4 days after cell separation, cell-killing capacity thereof against K562 cell line was compared. The K562 cell line was stained with 30 µM Calcein-AM (Invitrogen, C3099) according to the manufacturer's instructions, and then incubated with the NK-92MI cell line on a U-bottom plate at an E:T ratio of 10:1, 3:1, 1:1, or 0.3:1. After 4 hours, each incubate was taken out by 100 µL, and the amount of Calcein-AM secreted by cell death was measured with a fluorometer (VictorTMX3, PerkinElmer). As a result, as illustrated in Fig. 14, it was identified that the HLA-I positive cells and the HLA-I negative cells had equivalent cell-killing capacity against the K562 cell line. From these results, it was found that deletion of HLA-I did not affect the cell-killing capacity.

### Example 3. Preparation and identification of HLA-I- and HLA-II-deleted cells

### Example 3.1. Preparation and incubation of NK cells

Cryopreserved peripheral blood mononuclear cells (PBMCs) were rapidly dissolved in a water bath at 37°C, and then transferred to a 50-mL conical tube. With shaking of the tube, thawing media (RPMI, 11875-093 + 10% FBS + 55 µM β-ME) was added dropwise thereto and mixed. Subsequently, centrifugation was performed at 1,200 rpm for 10 minutes at 4°C to remove the supernatant, and resuspended in 10 mL of CellGro SCGM (CELLGENIX, 2001) media. Then, the number of cells was quantified. The cells were resuspended in culture media (CellGro SCGM + 10 ng/mL OKT3 + 500 IU/mL IL-2 + 5% Human plasma) at a concentration of 1 × 10⁶ cells/mL, and then placed in Culture Bag (NIPRO, 87-352). Incubation was performed in a CO₂ incubator at 37°C for 24 hours, and then transfection was performed.

### Example 3.2. Preparation of T cells and incubation method

Cryopreserved peripheral blood mononuclear cells (PBMCs) were rapidly dissolved in a water bath at 37°C, and then transferred to a 50-mL conical tube. With shaking of the tube, thawing media (RPMI, 11875-093 + 10% FBS + 55 µM β-ME) was added dropwise thereto and mixed. Subsequently, centrifugation was performed at 1,200 rpm for 10 minutes at 4°C to remove the supernatant, and resuspended in 40 mL of MACS buffer (PBS + 0.5% FBS + 2mM EDTA). Then, the number of cells was quantified. Treatment with 20 µL of CD3 microbeads (Miltenyi Biotec, 130-050-101) per 10⁷ cells was performed. Then, light was blocked for 15 minutes and incubated at 4°C. Subsequently, centrifugation was performed at 1,350 rpm for 8 minutes at 4°C to remove the supernatant, and the resultant was resuspended in 500 µL of MACS buffer. Then, the resuspension was loaded onto an LS column (Miltenyi Biotec, 130-042-401) mounted on QuadroMACS separator (Miltenyi Biotec, 130-090-976). The LS column was washed 3 times with MACS buffer, and removed from the QuadroMACS separator. Then, the removed LS column was pressed with a plunger to obtain CD3 positive cells. The cells were resuspended at a concentration of 1 × 10⁶ cells/mL in T-cell culture media (X-VIVO15 (Lonza, BE02-060Q) + 40 µL/mL Dynabeads Human T-Activator CD3/CD28 (Gibco, 111.3 ID) + 200 IU/mL IL-2 + 5% Human plasma) and then placed in Culture Bag (NIPRO, 87-352). Incubation was performed in a CO₂ incubator at 37°C for 24 hours, and then transfection was performed.

### Example 3.3. Preparation of HLA-deleted NK cells and T cells using selected gRNAs

37.5 µg of each of the gRNAs was incubated at 65°C for 10 minutes to form a single strand. Then, 37.5 µg of Cas9 protein (Clontech, M0646T) was added thereto and incubation was performed at 25°C for 10 minutes to prepare a Cas9-gRNA complex (RNP complex). In case of multiplex deletion, the sum of the amounts of respective gRNAs was set to 37.5 µg. The RNP complex was transfected into 2 × 10⁶ cells with 4D-Nucleofector™ X Unit (Lonza, AAF-1002X) using P3 Primary Cell 4D-Nucleofector® X Kit L (Lonza, V4XP-3024). The transfected cells were incubated for 3 days, and then production of cytokines was observed. The transfected cells were incubated for 14 days, and then it was identified, by flow cytometry, whether HLA expression was decreased.

### Example 3.4. Identification of decreased expression of HLA using flow cytometry

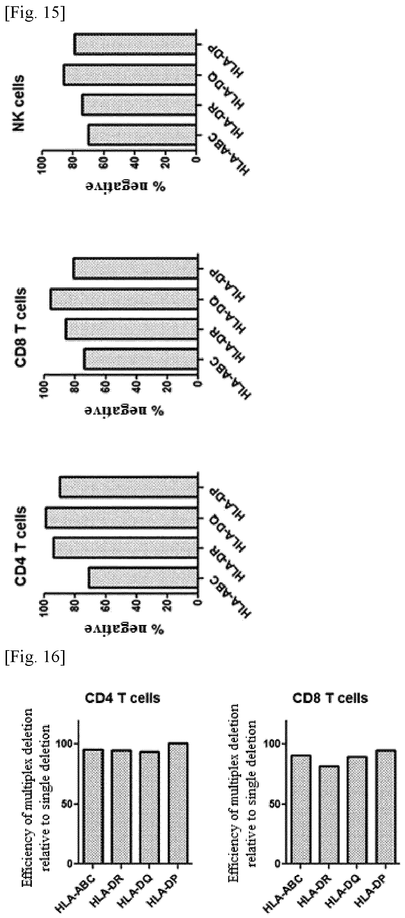

For each of the RNP complex-transfected cells and the control group cells, 2 × 10⁵ cells were suspended in 100 µL of FACS buffer (1% FBS/sheath buffer) and prepared in a 5-mL tube. Cell staining was carried out over 3 times. For primary staining, anti-HLA-DP (Abcam, ab20897) was used; for secondary staining, PE Goat anti-mouse IgG (eBioscience, 12-4010-82) was used; and for tertiary staining, V450 anti-CD4 (BD, 560345), APC-Cy7 anti-CD8 (BD, 557834), BV510 anti-HLA-ABC (Biolegend, 311436), PE-Cy7 anti-HLA-DR (eBioscience, 25-9952-42), Alexa647 anti-HLA-DQ (BD, 564806) were used. On the other hand, in case of NK cells, BV421 anti-CD56 (Biolegend, 318328) was used in place of V450 anti-CD4. Each time, after the antibody treatment, light was blocked for 30 minutes and incubation was performed at 4°C. Thereafter, 3 mL of FACS buffer was added thereto, and centrifugation was performed at 2,000 rpm for 3 minutes at 4°C to remove the supernatant. All stained samples were obtained and analyzed with LSR Fortessa. The results are illustrated in Figs. 15 to 17.

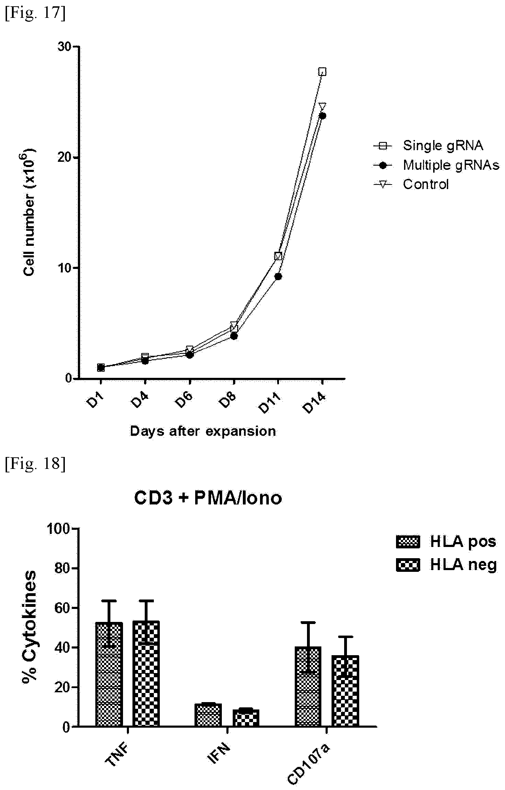

As gRNAs used to delete respective HLAs, B2M-01, DRA-20, DQA-14, and DPA-13 were used. From the results in Fig. 15, it was found that upon transfection with a single gRNA, deletion of the target HLA was achieved with high efficiency of at least 70% and up to 99%. From the results in Fig. 16, it was found that efficiency of the multiplex deletion was not remarkably decreased as compared with efficiency of the single deletion. When efficiency of the multiplex deletion was compared with respect to the single deletion, the efficiency was represented by multiplying a value, which was obtained by dividing the '% negative' value of the single deletion by the '% negative' value of the multiplex deletion, by 100. In addition, referring to the results in Fig. 17, a 14-day incubation rate for the RNP complex-transfected cells was found to be similar to that of the control group cells. In particular, it was identified that there was no difference in terms of incubation rate between single gRNA (DPA-13)-transfected cells and multiple gRNA-transfected cells.

### Example 3.5. Analysis of activity of HLA-deleted T cells and NK cells

For each of the RNP complex-transfected cells and the control cells, 1 × 10⁶ cells were subjected to treatment with PMA, ionomycin (Cell Stimulation Cocktail, eBioscience, 00-4970-03), and APC anti-CD107α (BD, 560664) followed by incubation, or were incubated with APC anti-CD107α and 2 × 10⁵ K562 cells. After 5 hours, treatment with PerCP-Cy5.5 anti-CD3 (Tonbo, 65-0038-T100), BV421 anti-CD56 (Biolegend, 318328), FITC anti-B2M (Biolegend, 316304), APC-Cy7 anti-HLA-ABC (Biolegend, 311426), and PE anti-HLA-DR/DP/DQ (Miltenyi Biotec, 130-104-827) was performed. Then, light was blocked for 30 minutes and incubation was performed at 4°C so as to carry out surface staining.

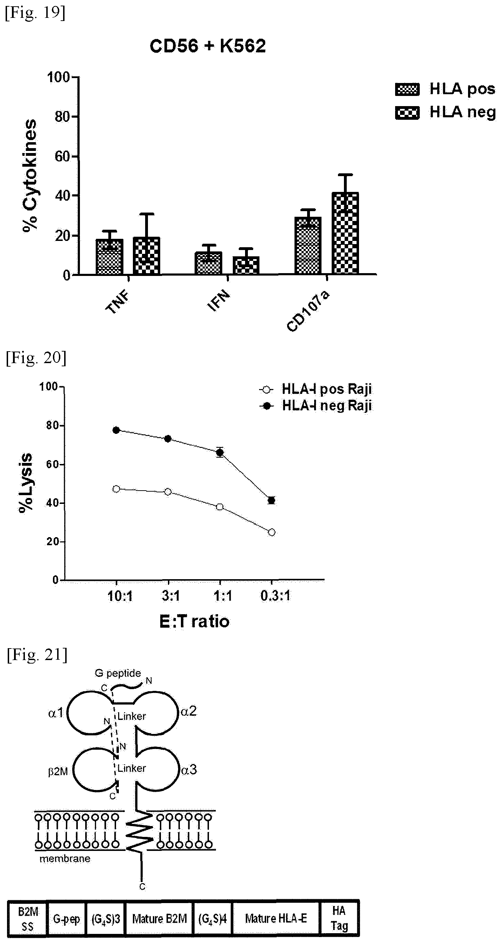

Thereafter, 3 mL of FACS buffer was added thereto, and centrifugation was performed at 2,000 rpm for 3 minutes at 4°C to remove the supernatant. Then, fixation and permeation were performed for 30 minutes using BD Cytofix/Cytoperm™ buffer (BD, 554722). Washing was performed twice with IX Perm/Wash buffer (BD, 554723), and treatment with PE-Cy7 anti-TNF-α (eBioscience, 25-7349-82) and V500 anti-IFN-y (BD, 554701) was performed. Light was blocked for 30 minutes and incubation was performed at 4°C so as to carry out intracellular staining. Subsequently, 3 mL of FACS buffer was added thereto, and centrifugation was performed at 2,000 rpm for 3 minutes at 4°C to remove the supernatant. Then, washing was performed twice with IX Perm/Wash buffer, and cytokine production in T cells and NK cells was analyzed with flow cytometry. The results are illustrated in Figs. 18 and 19.

In Fig. 18, it was identified that even when HLA was deleted, the amounts of TNF-α, IFN-γ, and CD 107α, which are secreted when T cells were activated, are not different from HLA positive cells. Also in Fig. 19, it was identified that even when HLA was deleted, the amounts of TNF-α, IFN-γ, and CD107α, which are secreted when NK cells are activated, were not different from HLA positive cells. From these results, it was found that activity of NK cells was maintained even when HLA-I and HLA-II were deleted.

### Example 4. Synthesis of HLA-E-expressing vector and identification of expression

### Example 4.1. Evaluation of cell-killing capacity of NK cells against HLA-I-deleted Raji cell line

In order to identify whether cell-killing capacity of NK cells is increased in HLA-I-deleted cells, Raji cell line was transfected with B2M-01 RNP complex, and HLA-I positive cells and HLA-I negative cells were separated using a cell separator. The respective cells were stained with Calcein-AM according to the manufacturer's instructions, and then 1 × 10⁴ cells were incubated with NK-92MI cell line on a U-bottom plate at an E:T ratio of 10:1, 3:1, 1:1, or 0.3:1. After 5 hours, the amount of Calcein-AM secreted by cell death was measured with a fluorometer. As illustrated in Fig. 20, it was identified that cell-killing capacity of NK cells was increased in HLA-I negative cells as compared with HLA-I positive cells.

### Example 4.2. Synthesis of HLA-E vector

In order to avoid a cell-killing phenomenon caused by NK cells which occurs when cells are transfected with B2M RNP complex so as to delete HLA-I, as in Example 4.1., an HLA-E vector for introducing HLA-E into the cells was synthesized. That is, transformed HLA-E (G-B2M-HLA-E) was synthesized in which B2M (SEQ ID NO: 237) was linked, via three first G₄S linkers (SEQ ID NO: 238), to G-peptide (SEQ ID NO: 236) connected to B2M signal peptide (B2M SS; SEQ ID NO: 235), and B2M was linked, via four second G₄S linkers (SEQ ID NO: 241), to HLA-E (SEQ ID NO: 240) attached with HA tag (SEQ ID NO: 239). The respective sequences are shown in Table 6 below. The synthesized transformed HLA-E was cloned by insertion into the pLVX-EF1α-IRES-Puro Vector (Clontech, 631988), and the structure thereof is as illustrated in Fig. 21.

**[Table 6]**

| Transformed HLA-E | Amino acid sequence |
|---|---|
| B2M SS | MSRSVALAVLALLSLSGLEA (SEQ ID NO: 235) |
| G-peptide | VMAPRTLFL (SEQ ID NO: 236) |
| B2M |  (SEQ ID NO: 237) |
| First linker (G₄S linker 1) | GGGGSGGGGSGGGGS (SEQ ID NO: 238) |
| HA tag | YPYDVPDYA (SEQ ID NO: 239) |
| HLA-E | GSHSLKYFHTSVSRPGRGEPRFISVGYVDDTQFVRFDNDAASP RMVPRAPWMEQEGSEYWDRETRSARDTAQIFRVNLRTLRGY YNQSEAGSHTLQWMHGCELGPDGRFLRGYEQFAYDGKDYLT LNEDLRSWTAVDTAAQISEQKSNDASEAEHQRAYLEDTCVE WLHKYLEKGKETLLHLEPPKTHVTHHPISDHEATLRCWALGF YPAEITLTWQQDGEGHTQDTELVETRPAGDGTFQKWAAVVV PSGEEQRYTCHVQHEGLPEPVTLRWKPASQPTIPIVGIIAGLVL LGSVVSGAVVAAVIWRKKSSGGKGGSYSKAEWSDSAQGSES HSL (SEQ ID NO: 240) |
| Second linker (G₄S linker 2) | GGGGSGGGGSGGGGSGGGGS (SEQ ID NO: 241) |

### Example 4.3. Expression of HLA-E through transduction into K562 cell line and identification thereof

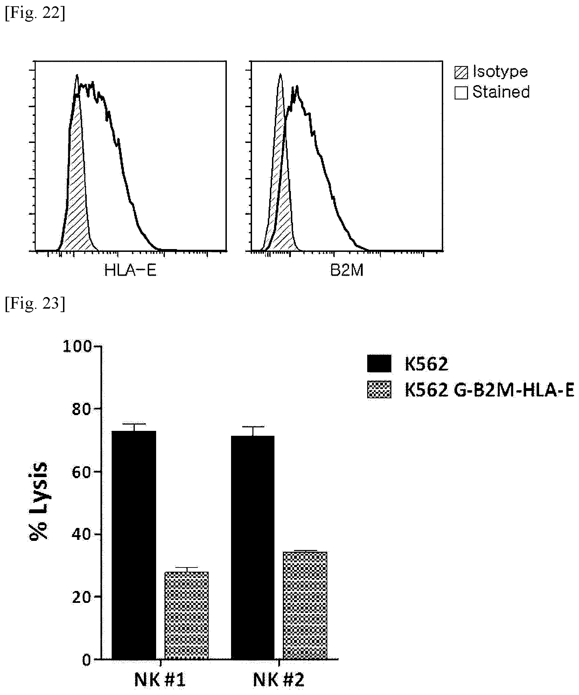

The transformed HLA-E-inserted pLVX-EFlα-IRES-Puro Vector was transfected into 293T cell line together with a lenti viral packaging vector. After 3 days, the lentiviral supernatant was obtained through a 0.45-µm filter. K562 cell line was subjected to treatment with the lentiviral supernatant, and centrifugation was performed at 3,000 rpm for 1 hour at 32°C. After 3 days, 1 × 10⁶ cells were transferred to a 5-mL tube, and cell surface staining was carried out for 30 minutes with PE-Cy7 anti-HLA-E (Biolegend, 342608) and APC anti-B2M (Biolegend, 316312). Washing with FACS buffer was performed, and then expression of HLA-E and B2M was checked with flow cytometry. As can be seen from Fig. 22, it was identified that HLA-E and B2M were expressed at high levels in the K562 cell line expressing the transformed HLA-E.

### Example 4.4. Evaluation of cell-killing capacity in HLA-E-introduced cells

Each of the K562 cell line expressing the transformed HLA-E and the control group K562 cell line was stained with Calcein-AM according to the manufacturer's instructions, and then 1 × 10⁴ cells were incubated with NK cells on a U-bottom plate at an E:T ratio of 10:1, 3:1, 1: 1, or 0.3:1. After 5 hours, 100 µL was taken out from each incubate, and the amount of Calcein-AM secreted by cell death was measured with a fluorometer (VictorTMX3, PerkinElmer).

As can be seen from Fig. 23, it was identified that a cell death phenomenon caused by NK cells was significantly decreased in case of the K562 cell line (K562 G-B2M-HLA-E) expressing the transformed HLA-E as compared with the control group K562 cell line (K562). From these results, it was found that expression of HLA-E could prevent cell death caused by NK cells.

### Example 5. Introduction of HLA-E to HLA-I- and HLA-II-deleted NK cells

HLA-E to which G-peptide was bound was introduced to the HLA-I- and HLA-II-deleted NK cells prepared in Example 3 using the HLA-E vector prepared as in Example 4.2, to prepare transformed NK cells.

## Claims

1. A guide RNA that complementarily binds to a nucleic acid sequence encoding β2-microglobulin (B2M), the guide RNA comprising any one nucleic acid sequence selected from the group consisting of SEQ ID NO: 1, SEQ ID NO: 6, SEQ ID NO: 17, and SEQ ID NO: 26.

2. A guide RNA that complementarily binds to a nucleic acid sequence encoding HLA-DQ, the guide RNA comprising any one nucleic acid sequence selected from the group consisting of SEQ ID NO: 64, SEQ ID NO: 65, SEQ ID NO: 87, and SEQ ID NO: 90.

3. A guide RNA that complementarily binds to a nucleic acid sequence encoding HLA-DP, the guide RNA comprising the nucleic acid sequence of SEQ ID NO: 123 or SEQ ID NO: 129.

4. A guide RNA that complementarily binds to a nucleic acid sequence encoding HLA-DR, the guide RNA comprising any one nucleic acid sequence selected from the group consisting of SEQ ID NO: 186, SEQ ID NO: 188, and SEQ ID NO: 225.

5. A composition for inhibiting gene expression comprising as active ingredients:
the guide RNA of any one of claims 1 to 4 or a nucleotide sequence encoding the guide RNA; and
an RNA-guided endonuclease or a nucleotide sequence encoding the RNA-guided endonuclease.

6. The composition of claim 5, wherein the RNA-guided endonuclease is any one selected from the group consisting of Cas1, Cas1B, Cas2, Cas3, Cas4, Cas5, Cas6, Cas7, Cas8, Cas9, Cas10, Cas12a, Cas12b, Cas12c, Cas12d, Cas12e, Cas 13a, Cas 13b, Cas 13c, Cas 13d, Cpf1, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, and Csf4.

7. A transformed cell in which expression of MHC I cell membrane receptor and MHC II cell membrane receptor is inhibited.

8. The transformed cell of claim 7, wherein the transformed cell expresses a peptide antigen on the cell membrane surface.

9. The transformed cell of claim 8, wherein the peptide antigen is G-peptide.

10. The transformed cell of claim 9, wherein the G-peptide is bound to a modified MHC I cell membrane receptor.

11. The transformed cell of claim 10, wherein the modified MHC I cell membrane receptor has a structure in which HLA-E and B2M are linked.

12. The transformed cell of claim 11, wherein the C-terminus of the B2M is linked, via a first linker, to the N-terminus of α1 of the HLA-E, and the C-terminus of the G-peptide is linked, via a second linker, to the N-terminus of the B2M in the modified MHC I cell membrane receptor.

13. The transformed cell of claim 12, wherein the G-peptide has the sequence of SEQ ID NO: 236.

14. The transformed cell of claim 12, wherein the HLA-E has the sequence of SEQ ID NO: 240.

15. The transformed cell of claim 12, wherein the B2M has the sequence of SEQ ID NO: 237.

16. The transformed cell of claim 12, wherein the first linker has the sequence of SEQ ID NO: 238.

17. The transformed cell of claim 12, wherein the second linker has the sequence of SEQ ID NO: 241.

18. The transformed cell of claim 7, wherein modification in a gene encoding the MHC I cell membrane receptor is performed using the guide RNA of claim 1.

19. The transformed cell of claim 7, wherein modification in DQ, DP, and DR genes encoding the MHC II cell membrane receptor is performed using the guide RNAs of claims 2, 3, and 4, respectively.

20. The transformed cell of claim 7, wherein the transformed cell is a therapeutic allogeneic cell.

21. The transformed cell of claim 20, wherein the therapeutic allogeneic cell is an immune cell or stem cell.

22. The transformed cell of claim 21, wherein the immune cell is an NK cell or T cell.

23. A pharmaceutical composition for treating cancer, an infectious disease, a degenerative disease, a hereditary disease, or an immune disease, comprising as an active ingredient the transformed cell of claim 7.

24. The pharmaceutical composition of claim 23, wherein the cancer is any one selected from the group consisting of chronic lymphocytic leukemia (CLL), B-cell acute lymphocytic leukemia (B-ALL), acute lymphoblastic leukemia, acute myeloid leukemia, lymphoma, non-Hodgkin's lymphoma (NHL), multiple myeloma, blood cancer, gastric cancer, liver cancer, pancreatic cancer, colorectal cancer, lung cancer, breast cancer, ovarian cancer, skin cancer, melanoma, sarcoma, prostate cancer, esophageal cancer, hepatocellular carcinoma, astrocytoma, mesothelioma, head and neck cancer, and medulloblastoma.

25. The pharmaceutical composition of claim 23, wherein the infectious disease is any one selected from the group consisting of hepatitis B, hepatitis C, human papilloma virus (HPV) infection, cytomegalovirus infection, Epstein Barr virus (EBV) infection, viral respiratory disease, and influenza.

26. A method for treating cancer, an infectious disease, a degenerative disease, a hereditary disease, or an immune disease, comprising administering to a subject, the pharmaceutical composition of any one of claims 23 to 25.

27. The method of claim 26, wherein the administration is performed via any one route selected from the group consisting of intravenous, intramuscular, intradermal, subcutaneous, intraperitoneal, intraarteriolar, intraventricular, intralesional, intrathecal, topical, and combinations thereof.

28. A use of a transformed cell for treating cancer, an infectious disease, a degenerative disease, a hereditary disease, or an immune disease, wherein expression of MHC I cell membrane receptor and MHC II cell membrane receptor is inhibited in the transformed cell, and the transformed cell expresses a peptide antigen bound to a modified MHC I cell membrane receptor on the cell membrane surface.


<110>    MOGAM INSTITUTE FOR BIOMEDICAL RESEARCH

<120>    AN ENGINEERED IMMUNE CELL AND USE THEREOF

<130>    PCB711063MOG

<150>    US 62/587,068
<151>    2017-11-16

<160>    259

<170>    KoPatentIn 3.0

<210>    1
<211>    20
<212>    RNA
<213>    Artificial Sequence

<220>
<223>    gRNA of B2M-01


<400>    1
gaguagcgcg agcacagcua                                                    20


<210>    2
<211>    20
<212>    RNA
<213>    Artificial Sequence

<220>
<223>    gRNA of B2M-03


<400>    2
cucgcgcuac ucucucuuuc                                                    20


<210>    3
<211>    20
<212>    RNA
<213>    Artificial Sequence

<220>
<223>    gRNA of B2M-04


<400>    3
gcauacucau cuuuuucagu                                                    20


<210>    4
<211>    20
<212>    RNA
<213>    Artificial Sequence

<220>
<223>    gRNA of B2M-05


<400>    4
gcuacucucu cuuucuggcc                                                    20

<210>    5
<211>    20
<212>    RNA
<213>    Artificial Sequence

<220>
<223>    gRNA of B2M-06


<400>    5
ggcauacuca ucuuuuucag                                                    20


<210>    6
<211>    20
<212>    RNA
<213>    Artificial Sequence

<220>
<223>    gRNA of B2M-07


<400>    6
ggccacggag cgagacaucu                                                    20


<210>    7
<211>    20
<212>    RNA
<213>    Artificial Sequence

<220>
<223>    gRNA of B2M-08


<400>    7
ggccgagaug ucucgcuccg                                                    20


<210>    8
<211>    20
<212>    RNA
<213>    Artificial Sequence

<220>
<223>    gRNA of B2M-09


<400>    8
ucacgucauc cagcagagaa                                                    20


<210>    9
<211>    20
<212>    RNA
<213>    Artificial Sequence

<220>
<223>    gRNA of B2M-10

<400>    9
acaaagucac augguucaca                                                    20


<210>    10
<211>    20
<212>    RNA
<213>    Artificial Sequence

<220>
<223>    gRNA of B2M-11


<400>    10
agucacaugg uucacacggc                                                    20


<210>    11
<211>    20
<212>    RNA
<213>    Artificial Sequence

<220>
<223>    gRNA of B2M-12


<400>    11
aagucaacuu caaugucgga                                                    20


<210>    12
<211>    20
<212>    RNA
<213>    Artificial Sequence

<220>
<223>    gRNA of B2M-13


<400>    12
cauacucauc uuuuucagug                                                    20


<210>    13
<211>    20
<212>    RNA
<213>    Artificial Sequence

<220>
<223>    gRNA of B2M-14


<400>    13
uccugaauug cuaugugucu                                                    20


<210>    14
<211>    20
<212>    RNA
<213>    Artificial Sequence

<220>
<223>    gRNA of B2M-15

<400>    14
cgugaguaaa ccugaaucuu                                                    20


<210>    15
<211>    20
<212>    RNA
<213>    Artificial Sequence

<220>
<223>    gRNA of B2M-16


<400>    15
uuggaguacc ugaggaauau                                                    20


<210>    16
<211>    20
<212>    RNA
<213>    Artificial Sequence

<220>
<223>    gRNA of B2M-17


<400>    16
aggguaggag agacucacgc                                                    20


<210>    17
<211>    20
<212>    RNA
<213>    Artificial Sequence

<220>
<223>    gRNA of B2M-18


<400>    17
acagcccaag auaguuaagu                                                    20


<210>    18
<211>    20
<212>    RNA
<213>    Artificial Sequence

<220>
<223>    gRNA of B2M-19


<400>    18
auacucaucu uuuucagugg                                                    20


<210>    19
<211>    20
<212>    RNA
<213>    Artificial Sequence

<220>
<223>    gRNA of B2M-20


<400>    19
uggaguaccu gaggaauauc                                                    20


<210>    20
<211>    20
<212>    RNA
<213>    Artificial Sequence

<220>
<223>    gRNA of B2M-21


<400>    20
aagaaaagga aacugaaaac                                                    20


<210>    21
<211>    20
<212>    RNA
<213>    Artificial Sequence

<220>
<223>    gRNA of B2M-22


<400>    21
aagaaggcau gcacuagacu                                                    20


<210>    22
<211>    20
<212>    RNA
<213>    Artificial Sequence

<220>
<223>    gRNA of B2M-23


<400>    22
acauguaagc agcaucaugg                                                    20


<210>    23
<211>    20
<212>    RNA
<213>    Artificial Sequence

<220>
<223>    gRNA of B2M-24


<400>    23
acccagacac auagcaauuc                                                    20


<210>    24
<211>    20
<212>    RNA

<213>    Artificial Sequence

<220>
<223>    gRNA of B2M-25


<400>    24
acuugucuuu cagcaaggac                                                    20


<210>    25
<211>    20
<212>    RNA
<213>    Artificial Sequence

<220>
<223>    gRNA of B2M-26


<400>    25
caagccagcg acgcagugcc                                                    20


<210>    26
<211>    20
<212>    RNA
<213>    Artificial Sequence

<220>
<223>    gRNA of B2M-27


<400>    26
cacagcccaa gauaguuaag                                                    20


<210>    27
<211>    20
<212>    RNA
<213>    Artificial Sequence

<220>
<223>    gRNA of B2M-29


<400>    27
caucacgaga cucuaagaaa                                                    20


<210>    28
<211>    20
<212>    RNA
<213>    Artificial Sequence

<220>
<223>    gRNA of B2M-30


<400>    28
cgcagugcca gguuagagag                                                    20


<210>    29

<211>    20
<212>    RNA
<213>    Artificial Sequence

<220>
<223>    gRNA of B2M-31


<400>    29
cuaaccuggc acugcgucgc                                                    20


<210>    30
<211>    20
<212>    RNA
<213>    Artificial Sequence

<220>
<223>    gRNA of B2M-32


<400>    30
gaaagucccu cucucuaacc                                                    20


<210>    31
<211>    20
<212>    RNA
<213>    Artificial Sequence

<220>
<223>    gRNA of B2M-33


<400>    31
gagacaugua agcagcauca                                                    20


<210>    32
<211>    20
<212>    RNA
<213>    Artificial Sequence

<220>
<223>    gRNA of B2M-34


<400>    32
gagucucgug auguuuaaga                                                    20


<210>    33
<211>    20
<212>    RNA
<213>    Artificial Sequence

<220>
<223>    gRNA of B2M-35


<400>    33
gcagugccag guuagagaga                                                    20

<210>    34
<211>    20
<212>    RNA
<213>    Artificial Sequence

<220>
<223>    gRNA of B2M-36


<400>    34
uaagaaggca ugcacuagac                                                    20


<210>    35
<211>    20
<212>    RNA
<213>    Artificial Sequence

<220>
<223>    gRNA of B2M-37


<400>    35
ucgaucuaug aaaaagacag                                                    20


<210>    36
<211>    20
<212>    RNA
<213>    Artificial Sequence

<220>
<223>    gRNA of B2M-39


<400>    36
uucagacuug ucuuucagca                                                    20


<210>    37
<211>    20
<212>    RNA
<213>    Artificial Sequence

<220>
<223>    gRNA of B2M-40


<400>    37
uuccugaauu gcuauguguc                                                    20


<210>    38
<211>    20
<212>    RNA
<213>    Artificial Sequence

<220>
<223>    gRNA of B2M-41


<400>    38

uaagaaaagg aaacugaaaa                                                    20

<210>    39
<211>    20
<212>    RNA
<213>    Artificial Sequence

<220>
<223>    gRNA of B2M-42

<400>    39
cuggcacugc gucgcuggcu                                                    20

<210>    40
<211>    20
<212>    RNA
<213>    Artificial Sequence

<220>
<223>    gRNA of B2M-43

<400>    40
ugcgucgcug gcuuggagac                                                    20

<210>    41
<211>    20
<212>    RNA
<213>    Artificial Sequence

<220>
<223>    gRNA of B2M-44

<400>    41
gcuggcuugg agacagguga                                                    20

<210>    42
<211>    20
<212>    RNA
<213>    Artificial Sequence

<220>
<223>    gRNA of B2M-45

<400>    42
agacagguga cggucccugc                                                    20

<210>    43
<211>    20
<212>    RNA
<213>    Artificial Sequence

<220>
<223>    gRNA of B2M-46

<400>    43
caaucaggac aaggcccgca                                                    20


<210>    44
<211>    20
<212>    RNA
<213>    Artificial Sequence

<220>
<223>    gRNA of B2M-47


<400>    44
ccugcgggcc uuguccugau                                                    20


<210>    45
<211>    20
<212>    RNA
<213>    Artificial Sequence

<220>
<223>    gRNA of B2M-48


<400>    45
ccaaucagga caaggcccgc                                                    20


<210>    46
<211>    20
<212>    RNA
<213>    Artificial Sequence

<220>
<223>    gRNA of B2M-49


<400>    46
cgggccuugu ccugauuggc                                                    20


<210>    47
<211>    20
<212>    RNA
<213>    Artificial Sequence

<220>
<223>    gRNA of B2M-50


<400>    47
gggccuuguc cugauuggcu                                                    20


<210>    48
<211>    20
<212>    RNA
<213>    Artificial Sequence

<220>

<223>    gRNA of B2M-51

<400>    48
gugcccagcc aaucaggaca                                                    20

<210>    49
<211>    20
<212>    RNA
<213>    Artificial Sequence

<220>
<223>    gRNA of B2M-52

<400>    49
aaacgcgugc ccagccaauc                                                    20

<210>    50
<211>    20
<212>    RNA
<213>    Artificial Sequence

<220>
<223>    gRNA of B2M-53

<400>    50
gggcacgcgu uuaauauaag                                                    20

<210>    51
<211>    20
<212>    RNA
<213>    Artificial Sequence

<220>
<223>    gRNA of B2M-54

<400>    51
cacgcguuua auauaagugg                                                    20

<210>    52
<211>    20
<212>    RNA
<213>    Artificial Sequence

<220>
<223>    gRNA of B2M-55

<400>    52
uauaagugga ggcgucgcgc                                                    20

<210>    53
<211>    20
<212>    RNA
<213>    Artificial Sequence

<220>
<223>    gRNA of B2M-56


<400>    53
aaguggaggc gucgcgcugg                                                    20


<210>    54
<211>    20
<212>    RNA
<213>    Artificial Sequence

<220>
<223>    gRNA of B2M-57


<400>    54
aguggaggcg ucgcgcuggc                                                    20


<210>    55
<211>    20
<212>    RNA
<213>    Artificial Sequence

<220>
<223>    gRNA of B2M-58


<400>    55
uuccugaagc ugacagcauu                                                    20


<210>    56
<211>    20
<212>    RNA
<213>    Artificial Sequence

<220>
<223>    gRNA of B2M-59


<400>    56
uccugaagcu gacagcauuc                                                    20


<210>    57
<211>    20
<212>    RNA
<213>    Artificial Sequence

<220>
<223>    gRNA of B2M-60


<400>    57
ugggcuguga caaagucaca                                                    20


<210>    58
<211>    20

<212>    RNA
<213>    Artificial Sequence

<220>
<223>    gRNA of B2M-61


<400>    58
acucucucuu ucuggccugg                                                    20


<210>    59
<211>    20
<212>    RNA
<213>    Artificial Sequence

<220>
<223>    gRNA of DQA-08


<400>    59                                                               20
uuaggaucau ccucuuccca


<210>    60
<211>    20
<212>    RNA
<213>    Artificial Sequence

<220>
<223>    gRNA of DQA-09


<400>    60                                                               20
aacucuaccg cugcuaccaa


<210>    61
<211>    20
<212>    RNA
<213>    Artificial Sequence

<220>
<223>    gRNA of DQA-10


<400>    61                                                               20
acaaugucuu caccuccaca


<210>    62
<211>    20
<212>    RNA
<213>    Artificial Sequence

<220>
<223>    gRNA of DQA-11


<400>    62                                                               20
accaccguga ugagccccug

<210>    63
<211>    20
<212>    RNA
<213>    Artificial Sequence

<220>
<223>    gRNA of DQA-12


<400>    63                                                                    20
acccagugucacgggagacu


<210>    64
<211>    20
<212>    RNA
<213>    Artificial Sequence

<220>
<223>    gRNA of DQA-14


<400>    64                                                                    20
accuccacag gggcucauca

<210>    65
<211>    20
<212>    RNA
<213>    Artificial Sequence

<220>
<223>    gRNA of DQA-15


<400>    65                                                                    20
caaugucuucaccuccacag


<210>    66
<211>    20
<212>    RNA
<213>    Artificial Sequence

<220>
<223>    gRNA of DQA-16


<400>    66                                                                    20
cacaaugucuucaccuccac

<210>    67
<211>    20
<212>    RNA
<213>    Artificial Sequence

<220>
<223>    gRNA of DQA-17


<400>    67                                                                    20
caguacacccaugaauuuga

<210>	68
<211>	20
<212>	RNA
<213>	Artificial Sequence

<220>
<223>	gRNA of DQA-18


<400>	68	20
cucugugagcucugacauag


<210>	69
<211>	20
<212>	RNA
<213>	Artificial Sequence

<220>
<223>	gRNA of DQA-19


<400>	69	20
cuguggaggugaagacauug


<210>	70
<211>	20
<212>	RNA
<213>	Artificial Sequence

<220>
<223>	gRNA of DQA-20


<400>	70	20
ggcuggaaucucaggcucug


<210>	71
<211>	20
<212>	RNA
<213>	Artificial Sequence

<220>
<223>	gRNA of DQA-21


<400>	71	20
guugggcugacccaguguca


<210>	72
<211>	20
<212>	RNA
<213>	Artificial Sequence

<220>
<223>	gRNA of DQA-22


<400>	72	20
ucauggguguacuggccaga

<210>    73
<211>    20
<212>    RNA
<213>    Artificial Sequence

<220>
<223>    gRNA of DQA-23


<400>    73                                                                    20
uccaagucucccgugacacu


<210>    74
<211>    20
<212>    RNA
<213>    Artificial Sequence

<220>
<223>    gRNA of DQA-24


<400>    74                                                                    20
uccacaggggcucaucacgg


<210>    75
<211>    20
<212>    RNA
<213>    Artificial Sequence

<220>
<223>    gRNA of DQA-25


<400>    75                                                                    20
uguggaggugaagacauugu


<210>    76
<211>    20
<212>    RNA
<213>    Artificial Sequence

<220>
<223>    gRNA of DQA-26


<400>    76                                                                    20
uuccaagucucccgugacac


<210>    77
<211>    20
<212>    RNA
<213>    Artificial Sequence

<220>
<223>    gRNA of DQA-27

<400>    77                                                    20
uugggcugacccagugucac


<210>    78
<211>    20
<212>    RNA
<213>    Artificial Sequence

<220>
<223>    gRNA of DQA-28


<400>    78                                                    20
aacaucacauggcugagcaa

<210>    79
<211>    20
<212>    RNA
<213>    Artificial Sequence

<220>
<223>    gRNA of DQA-29


<400>    79                                                    20
acaucacauggcugagcaau


<210>    80
<211>    20
<212>    RNA
<213>    Artificial Sequence

<220>
<223>    gRNA of DQA-30


<400>    80                                                    20
agccaugugauguugaccac


<210>    81
<211>    20
<212>    RNA
<213>    Artificial Sequence

<220>
<223>    gRNA of DQA-31


<400>    81                                                    20
aggaaugaucacucuuggag


<210>    82
<211>    20
<212>    RNA
<213>    Artificial Sequence

<220>
<223>    gRNA of DQA-32

<400>    82                                                                    20
aucacucuuggagaggaagc


<210>    83
<211>    20
<212>    RNA
<213>    Artificial Sequence

<220>
<223>    gRNA of DQA-33


<400>    83                                                                    20
augacugcaagguggagcac


<210>    84
<211>    20
<212>    RNA
<213>    Artificial Sequence

<220>
<223>    gRNA of DQA-34


<400>    84                                                                    20
caagguggagcacuggggcc


<210>    85
<211>    20
<212>    RNA
<213>    Artificial Sequence

<220>
<223>    gRNA of DQA-35


<400>    85                                                                    20
caucaaauucauggguguac


<210>    86
<211>    20
<212>    RNA
<213>    Artificial Sequence

<220>
<223>    gRNA of DQA-36


<400>    86                                                                    20
caugugauguugaccacagg


<210>    87
<211>    20
<212>    RNA
<213>    Artificial Sequence

<220>

<223>    gRNA of DQA-37

<400>    87                                                                    20
ccucaccacagagguuccug

<210>    88
<211>    20
<212>    RNA
<213>    Artificial Sequence

<220>
<223>    gRNA of DQA-38

<400>    88                                                                    20
cucaucuccaucaaauucau

<210>    89
<211>    20
<212>    RNA
<213>    Artificial Sequence

<220>
<223>    gRNA of DQA-39

<400>    89                                                                    20
cucccugugggucaacaucaca

<210>    90
<211>    20
<212>    RNA
<213>    Artificial Sequence

<220>
<223>    gRNA of DQA-40

<400>    90                                                                    20
gaagaaggaaugaucacucu

<210>    91
<211>    20
<212>    RNA
<213>    Artificial Sequence

<220>
<223>    gRNA of DQA-41

<400>    91                                                                    20
gacugcaagguggagcacug

<210>    92
<211>    20
<212>    RNA
<213>    Artificial Sequence

<220>
<223>    gRNA of DQA-42

<400>    92                                                                    20
gagguaacugaucuugaaga

<210>    93
<211>    20
<212>    RNA
<213>    Artificial Sequence

<220>
<223>    gRNA of DQA-43

<400>    93                                                                    20
ggacaacaucuuuccuccug

<210>    94
<211>    20
<212>    RNA
<213>    Artificial Sequence

<220>
<223>    gRNA of DQA-44

<400>    94                                                                    20
gugcuguuuccucaccacag

<210>    95
<211>    20
<212>    RNA
<213>    Artificial Sequence

<220>
<223>    gRNA of DQA-45

<400>    95                                                                    20
ucuucugaaacacuggggua

<210>    96
<211>    20
<212>    RNA
<213>    Artificial Sequence

<220>
<223>    gRNA of DQA-47

<400>    96                                                                    20
uucauggguguacuggccag

<210>    97
<211>    20

<212>    RNA
<213>    Artificial Sequence

<220>
<223>    gRNA of DQA-48


<400>    97                                                                    20
agagacugugggucugcgccc


<210>    98
<211>    20
<212>    RNA
<213>    Artificial Sequence

<220>
<223>    gRNA of DQA-49


<400>    98                                                                    20
gacauaggggcuggaaucuc


<210>    99
<211>    20
<212>    RNA
<213>    Artificial Sequence

<220>
<223>    gRNA of DQA-50


<400>    99                                                                    20
gagacugugggucugcgcccu


<210>    100
<211>    20
<212>    RNA
<213>    Artificial Sequence

<220>
<223>    gRNA of DQA-51


<400>    100                                                                   20
ggccucgugggcauugugggu


<210>    101
<211>    20
<212>    RNA
<213>    Artificial Sequence

<220>
<223>    gRNA of DQA-52


<400>    101                                                                   20
gggccucgugggcauugugg

<210>    102
<211>    20
<212>    RNA
<213>    Artificial Sequence

<220>
<223>    gRNA of DQA-53

<400>    102                                                                    20
gucagagcucacagagacug

<210>    103
<211>    20
<212>    RNA
<213>    Artificial Sequence

<220>
<223>    gRNA of DQA-54

<400>    103                                                                    20
gucucugugagcucugacau

<210>    104
<211>    20
<212>    RNA
<213>    Artificial Sequence

<220>
<223>    gRNA of DQA-55

<400>    104                                                                    20
gugagcucugacauaggggc

<210>    105
<211>    20
<212>    RNA
<213>    Artificial Sequence

<220>
<223>    gRNA of DQA-56

<400>    105                                                                    20
guuggugcuuccagacacca

<210>    106
<211>    20
<212>    RNA
<213>    Artificial Sequence

<220>
<223>    gRNA of DQA-57

<400>    106                                                                    20
ucucugugagcucugacaua

<210>    107
<211>    20
<212>    RNA
<213>    Artificial Sequence

<220>
<223>    gRNA of DQA-58


<400>    107                                                                    20
ugacugcaagguggagcacu


<210>    108
<211>    20
<212>    RNA
<213>    Artificial Sequence

<220>
<223>    gRNA of DQA-59


<400>    108                                                                    20
ugcccaccacaaugcccacg


<210>    109
<211>    20
<212>    RNA
<213>    Artificial Sequence

<220>
<223>    gRNA of DQA-60


<400>    109                                                                    20
uggaagcaccaacugaacgc


<210>    110
<211>    20
<212>    RNA
<213>    Artificial Sequence

<220>
<223>    gRNA of DQA-61


<400>    110                                                                    20
ugugggccucgugggcauug


<210>    111
<211>    20
<212>    RNA
<213>    Artificial Sequence

<220>
<223>    gRNA of DQA-62

<400>    111                                                                    20
uuaccccaguguuucagaag


<210>    112
<211>    20
<212>    RNA
<213>    Artificial Sequence

<220>
<223>    gRNA of DQA-63


<400>    112                                                                    20
uuggaaaacacugugaccuc


<210>    113
<211>    20
<212>    RNA
<213>    Artificial Sequence

<220>
<223>    gRNA of DQA-64


<400>    113                                                                    20
uuggugcuuccagacaccaa


<210>    114
<211>    20
<212>    RNA
<213>    Artificial Sequence

<220>
<223>    gRNA of DQA-65


<400>    114                                                                    20
aaacaaagcucugcugcugg


<210>    115
<211>    20
<212>    RNA
<213>    Artificial Sequence

<220>
<223>    gRNA of DQA-66


<400>    115                                                                    20
aaaucucaucagcagaaggg


<210>    116
<211>    20
<212>    RNA
<213>    Artificial Sequence

<220>
<223>    gRNA of DQA-67

<400>    116                                                                    20
cuaaacaaagcucugcugcu


<210>    117
<211>    20
<212>    RNA
<213>    Artificial Sequence

<220>
<223>    gRNA of DPA-01


<400>    117                                                                    20
ucuaugcgucuguacaaacg


<210>    118
<211>    20
<212>    RNA
<213>    Artificial Sequence

<220>
<223>    gRNA of DPA-02


<400>    118                                                                    20
guacagacgcauagaccaac


<210>    119
<211>    20
<212>    RNA
<213>    Artificial Sequence

<220>
<223>    gRNA of DPA-03


<400>    119                                                                    20
uacagacgcauagaccaaca


<210>    120
<211>    20
<212>    RNA
<213>    Artificial Sequence

<220>
<223>    gRNA of DPA-04


<400>    120                                                                    20
gaaggagaccgucuggcauc


<210>    121
<211>    20
<212>    RNA
<213>    Artificial Sequence

<220>
<223>    gRNA of DPA-05


<400>    121                                                                    20
ggagaccgucuggcaucugg


<210>    122
<211>    20
<212>    RNA
<213>    Artificial Sequence

<220>
<223>    gRNA of DPA-06


<400>    122                                                                    20
gucuggcaucuggaggaguu


<210>    123
<211>    20
<212>    RNA
<213>    Artificial Sequence

<220>
<223>    gRNA of DPA-07


<400>    123                                                                    20
gugguuggaacgcuggauca


<210>    124
<211>    20
<212>    RNA
<213>    Artificial Sequence

<220>
<223>    gRNA of DPA-08


<400>    124                                                                    20
gucuucagggcgcauguugu


<210>    125
<211>    20
<212>    RNA
<213>    Artificial Sequence

<220>
<223>    gRNA of DPA-09


<400>    125                                                                    20
ugucuucagggcgcauguug


<210>    126
<211>    20
<212>    RNA

<213>    Artificial Sequence

<220>
<223>    gRNA of DPA-10


<400>    126                                                                    20
ucuucagggcgcauguugug


<210>    127
<211>    20
<212>    RNA
<213>    Artificial Sequence

<220>
<223>    gRNA of DPA-11


<400>    127                                                                    20
guugcauaccccagugcuug


<210>    128
<211>    20
<212>    RNA
<213>    Artificial Sequence

<220>
<223>    gRNA of DPA-12


<400>    128                                                                    20
gaccuuugugcccucagcag


<210>    129
<211>    20
<212>    RNA
<213>    Artificial Sequence

<220>
<223>    gRNA of DPA-13


<400>    129                                                                    20
gagacucagcaggaaagcca


<210>    130
<211>    20
<212>    RNA
<213>    Artificial Sequence

<220>
<223>    gRNA of DPA-14


<400>    130                                                                    20
gagccucaaaggaaaaggcu


<210>    131

<211>    20
<212>    RNA
<213>    Artificial Sequence

<220>
<223>    gRNA of DPA-15


<400>    131                                                                    20
gaucuugagagcccucuccu


<210>    132
<211>    20
<212>    RNA
<213>    Artificial Sequence

<220>
<223>    gRNA of DPA-16


<400>    132                                                                    20
gccaucaagggugagugcuc


<210>    133
<211>    20
<212>    RNA
<213>    Artificial Sequence

<220>
<223>    gRNA of DPA-17


<400>    133                                                                    20
gccaugacccccgggcccag


<210>    134
<211>    20
<212>    RNA
<213>    Artificial Sequence

<220>
<223>    gRNA of DPA-18


<400>    134                                                                    20
gcccagcuccacaggcuccu


<210>    135
<211>    20
<212>    RNA
<213>    Artificial Sequence

<220>
<223>    gRNA of DPA-19


<400>    135                                                                    20
gcccugagccucaaaggaaa

<210>    136
<211>    20
<212>    RNA
<213>    Artificial Sequence

<220>
<223>    gRNA of DPA-20


<400>    136                                                                    20
gccuuuuccuuugaggcuca


<210>    137
<211>    20
<212>    RNA
<213>    Artificial Sequence

<220>
<223>    gRNA of DPA-21


<400>    137                                                                    20
gcguucuggccaugaccccc


<210>    138
<211>    20
<212>    RNA
<213>    Artificial Sequence

<220>
<223>    gRNA of DPA-22


<400>    138                                                                    20
gcuuuccugcugagucuccg


<210>    139
<211>    20
<212>    RNA
<213>    Artificial Sequence

<220>
<223>    gRNA of DPA-23


<400>    139                                                                    20
ggaaacacggucaccucagg


<210>    140
<211>    20
<212>    RNA
<213>    Artificial Sequence

<220>
<223>    gRNA of DPA-24


<400>    140                                                                    20

ggacuucuaugacugcaggg

<210> 141
<211> 20
<212> RNA
<213> Artificial Sequence

<220>
<223> gRNA of DPA-25

<400> 141 20
ggagacugugcucugugccc

<210> 142
<211> 20
<212> RNA
<213> Artificial Sequence

<220>
<223> gRNA of DPA-26

<400> 142 20
ggccaugacccccgggccca

<210> 143
<211> 20
<212> RNA
<213> Artificial Sequence

<220>
<223> gRNA of DPA-27

<400> 143 20
ggccuagucggcaucaucgu

<210> 144
<211> 20
<212> RNA
<213> Artificial Sequence

<220>
<223> gRNA of DPA-29

<400> 144 20
gggaaacacggucaccucag

<210> 145
<211> 20
<212> RNA
<213> Artificial Sequence

<220>
<223> gRNA of DPA-30

<400>    145                                                                    20
gggccuagucggcaucaucg


<210>    146
<211>    20
<212>    RNA
<213>    Artificial Sequence

<220>
<223>    gRNA of DPA-31


<400>    146                                                                    20
gucauagaaguccucugcug


<210>    147
<211>    20
<212>    RNA
<213>    Artificial Sequence

<220>
<223>    gRNA of DPA-32


<400>    147                                                                    20
guccucugcugagggcacaa


<210>    148
<211>    20
<212>    RNA
<213>    Artificial Sequence

<220>
<223>    gRNA of DPA-33


<400>    148                                                                    20
guggaagcuguaaucuguuc


<210>    149
<211>    20
<212>    RNA
<213>    Artificial Sequence

<220>
<223>    gRNA of DPA-34


<400>    149                                                                    20
gugggaagaacuugucaaug


<210>    150
<211>    20
<212>    RNA
<213>    Artificial Sequence

<220>

<223>    gRNA of DPA-35

<400>    150                                                                    20
guugguggccugagugugguu

<210>    151
<211>    20
<212>    RNA
<213>    Artificial Sequence

<220>
<223>    gRNA of DPA-36

<400>    151                                                                    20
guugucucaggcaucuggau

<210>    152
<211>    20
<212>    RNA
<213>    Artificial Sequence

<220>
<223>    gRNA of DPA-37

<400>    152                                                                    20
gcugagucuccgaggagcug

<210>    153
<211>    20
<212>    RNA
<213>    Artificial Sequence

<220>
<223>    gRNA of DPA-38

<400>    153                                                                    20
ucucuacugucuuuaugcag

<210>    154
<211>    20
<212>    RNA
<213>    Artificial Sequence

<220>
<223>    gRNA of DPA-39

<400>    154                                                                    20
uauggaacauucugucuuca

<210>    155
<211>    20
<212>    RNA
<213>    Artificial Sequence

<220>
<223>    gRNA of DPA-40


<400>    155                                                                    20
ucaagaucacagcucugaua


<210>    156
<211>    20
<212>    RNA
<213>    Artificial Sequence

<220>
<223>    gRNA of DPA-41


<400>    156                                                                    20
ucaaacauaaacuccccugu


<210>    157
<211>    20
<212>    RNA
<213>    Artificial Sequence

<220>
<223>    gRNA of DPA-42


<400>    157                                                                    20
uaccguugguggccugagug


<210>    158
<211>    20
<212>    RNA
<213>    Artificial Sequence

<220>
<223>    gRNA of DPA-43


<400>    158                                                                    20
uccugagcacucacccuuga


<210>    159
<211>    20
<212>    RNA
<213>    Artificial Sequence

<220>
<223>    gRNA of DPA-44


<400>    159                                                                    20
ugaggugaccguguuuccca


<210>    160
<211>    20

<212>    RNA
<213>    Artificial Sequence

<220>
<223>    gRNA of DPA-45


<400>    160                                                                    20
ugcguucuggccaugacccc


<210>    161
<211>    20
<212>    RNA
<213>    Artificial Sequence

<220>
<223>    gRNA of DPA-46


<400>    161                                                                    20
uuuccuuugaggcucagggc


<210>    162
<211>    20
<212>    RNA
<213>    Artificial Sequence

<220>
<223>    gRNA of DPA-47


<400>    162                                                                    20
ugccgacuaggcccagcacc


<210>    163
<211>    20
<212>    RNA
<213>    Artificial Sequence

<220>
<223>    gRNA of DPA-48


<400>    163                                                                    20
ucagcaggaaagccaaggag


<210>    164
<211>    20
<212>    RNA
<213>    Artificial Sequence

<220>
<223>    gRNA of DPA-49


<400>    164                                                                    20
ugaagaugagauguucuaug

<210>    165
<211>    20
<212>    RNA
<213>    Artificial Sequence

<220>
<223>    gRNA of DPA-50


<400>    165                                                                    20
ugcugagucuccgaggagcu


<210>    166
<211>    20
<212>    RNA
<213>    Artificial Sequence

<220>
<223>    gRNA of DPA-51


<400>    166                                                                    20
ugagauguucuauguggauc


<210>    167
<211>    20
<212>    RNA
<213>    Artificial Sequence

<220>
<223>    gRNA of DPA-52


<400>    167                                                                    20
ugggaaacacggucaccuca


<210>    168
<211>    20
<212>    RNA
<213>    Artificial Sequence

<220>
<223>    gRNA of DPA-53


<400>    168                                                                    20
uggaagcuguaaucuguucu


<210>    169
<211>    20
<212>    RNA
<213>    Artificial Sequence

<220>
<223>    gRNA of DPA-54


<400>    169                                                                    20
uggacaagaaggagaccguc

<210>    170
<211>    20
<212>    RNA
<213>    Artificial Sequence

<220>
<223>    gRNA of DPA-55


<400>    170                                                                    20
ugcccacgaugaugccgacu


<210>    171
<211>    20
<212>    RNA
<213>    Artificial Sequence

<220>
<223>    gRNA of DPA-56


<400>    171                                                                    20
uggccaagccuuuuccuuug


<210>    172
<211>    20
<212>    RNA
<213>    Artificial Sequence

<220>
<223>    gRNA of DPA-57


<400>    172                                                                    20
guggcugugcaacggggagc


<210>    173
<211>    20
<212>    RNA
<213>    Artificial Sequence

<220>
<223>    gRNA of DPA-58


<400>    173                                                                    20
uccccugggcccgggggguca


<210>    174
<211>    20
<212>    RNA
<213>    Artificial Sequence

<220>
<223>    gRNA of DPA-59

<400>    174                                                                    20
ucaccucaggggggaucugga

<210>    175
<211>    20
<212>    RNA
<213>    Artificial Sequence

<220>
<223>    gRNA of DPA-60

<400>    175                                                                    20
ucuccuuccagaucccccug

<210>    176
<211>    20
<212>    RNA
<213>    Artificial Sequence

<220>
<223>    gRNA of DRA-08

<400>    176                                                                    20
aagaagaaaauggccauaag

<210>    177
<211>    20
<212>    RNA
<213>    Artificial Sequence

<220>
<223>    gRNA of DRA-09

<400>    177                                                                    20
aaucaugggcuaucaaaggu

<210>    178
<211>    20
<212>    RNA
<213>    Artificial Sequence

<220>
<223>    gRNA of DRA-10

<400>    178                                                                    20
agcugugcugaugagcgcuc

<210>    179
<211>    20
<212>    RNA
<213>    Artificial Sequence

<220>
<223>    gRNA of DRA-11

<400>    179                                                                    20
auaaguggagucccugugcu


<210>    180
<211>    20
<212>    RNA
<213>    Artificial Sequence

<220>
<223>    gRNA of DRA-12


<400>    180                                                                    20
acuuauggccauuuucuucu


<210>    181
<211>    20
<212>    RNA
<213>    Artificial Sequence

<220>
<223>    gRNA of DRA-13


<400>    181                                                                    20
augaugaaaaauccuagcac


<210>    182
<211>    20
<212>    RNA
<213>    Artificial Sequence

<220>
<223>    gRNA of DRA-14


<400>    182                                                                    20
cagagcgcccaagaagaaaa


<210>    183
<211>    20
<212>    RNA
<213>    Artificial Sequence

<220>
<223>    gRNA of DRA-15


<400>    183                                                                    20
caggaaucaugggcuaucaa


<210>    184
<211>    20
<212>    RNA
<213>    Artificial Sequence

<220>
<223>    gRNA of DRA-16


<400>    184                                                                    20
cuuauggccauuuucuucuu


<210>    185
<211>    20
<212>    RNA
<213>    Artificial Sequence

<220>
<223>    gRNA of DRA-17


<400>    185                                                                    20
gacugucucugacacuccug


<210>    186
<211>    20
<212>    RNA
<213>    Artificial Sequence

<220>
<223>    gRNA of DRA-18


<400>    186                                                                    20
gagccucuucucaagcacug


<210>    187
<211>    20
<212>    RNA
<213>    Artificial Sequence

<220>
<223>    gRNA of DRA-19


<400>    187                                                                    20
gauaguggaacuugcggaaa


<210>    188
<211>    20
<212>    RNA
<213>    Artificial Sequence

<220>
<223>    gRNA of DRA-20


<400>    188                                                                    20
gaugagcgcucaggaaucau


<210>    189
<211>    20
<212>    RNA

<213>    Artificial Sequence

<220>
<223>    gRNA of DRA-21


<400>    189                                                                    20
gcuaucaaagguaggugcug


<210>    190
<211>    20
<212>    RNA
<213>    Artificial Sequence

<220>
<223>    gRNA of DRA-22


<400>    190                                                                    20
guuaccucuggagguacugg


<210>    191
<211>    20
<212>    RNA
<213>    Artificial Sequence

<220>
<223>    gRNA of DRA-23


<400>    191                                                                    20
uagcacagggacuccacuua


<210>    192
<211>    20
<212>    RNA
<213>    Artificial Sequence

<220>
<223>    gRNA of DRA-24


<400>    192                                                                    20
ugaugaaaaauccuagcaca


<210>    193
<211>    20
<212>    RNA
<213>    Artificial Sequence

<220>
<223>    gRNA of DRA-25


<400>    193                                                                    20
ugaugagcgcucaggaauca


<210>    194

<211>    20
<212>    RNA
<213>    Artificial Sequence

<220>
<223>    gRNA of DRA-27


<400>    194                                                          20
uuugccagcuuugaggcuca


<210>    195
<211>    20
<212>    RNA
<213>    Artificial Sequence

<220>
<223>    gRNA of DRA-28


<400>    195                                                          20
aacuauacuccgaucaccaa


<210>    196
<211>    20
<212>    RNA
<213>    Artificial Sequence

<220>
<223>    gRNA of DRA-29


<400>    196                                                          20
agaagaacaugugaucaucc


<210>    197
<211>    20
<212>    RNA
<213>    Artificial Sequence

<220>
<223>    gRNA of DRA-30


<400>    197                                                          20
agcagagagggagguaccau


<210>    198
<211>    20
<212>    RNA
<213>    Artificial Sequence

<220>
<223>    gRNA of DRA-31


<400>    198                                                          20
agcgcuuugucaugauuucc

<210>    199
<211>    20
<212>    RNA
<213>    Artificial Sequence

<220>
<223>    gRNA of DRA-32


<400>    199                                                                    20
agcuguggacaaagccaacc


<210>    200
<211>    20
<212>    RNA
<213>    Artificial Sequence

<220>
<223>    gRNA of DRA-33


<400>    200                                                                    20
agggagguaccauuggugau


<210>    201
<211>    20
<212>    RNA
<213>    Artificial Sequence

<220>
<223>    gRNA of DRA-34


<400>    201                                                                    20
auaaacucgccugauugguc



<210>    202
<211>    20
<212>    RNA
<213>    Artificial Sequence

<220>
<223>    gRNA of DRA-35


<400>    202                                                                    20
auuggugaucggaguauagu


<210>    203
<211>    20
<212>    RNA
<213>    Artificial Sequence

<220>
<223>    gRNA of DRA-36

<400>    203                                                                    20
ccauguggauauggcaaaga

<210>    204
<211>    20
<212>    RNA
<213>    Artificial Sequence

<220>
<223>    gRNA of DRA-37

<400>    204                                                                    20
cuuugaggcucaaggugcau

<210>    205
<211>    20
<212>    RNA
<213>    Artificial Sequence

<220>
<223>    gRNA of DRA-38

<400>    205                                                                    20
cuuugucaugauuuccaggu

<210>    206
<211>    20
<212>    RNA
<213>    Artificial Sequence

<220>
<223>    gRNA of DRA-39

<400>    206                                                                    20
ggauauggcaaagaaggaga

<210>    207
<211>    20
<212>    RNA
<213>    Artificial Sequence

<220>
<223>    gRNA of DRA-40

<400>    207                                                                    20
uaucugaauccugaccaauc

<210>    208
<211>    20
<212>    RNA
<213>    Artificial Sequence

<220>
<223>    gRNA of DRA-41

<400>    208                                                                    20
ugagauuuuccauguggaua


<210>    209
<211>    20
<212>    RNA
<213>    Artificial Sequence

<220>
<223>    gRNA of DRA-42


<400>    209                                                                    20
ugaucacauguucuucugaa


<210>    210
<211>    20
<212>    RNA
<213>    Artificial Sequence

<220>
<223>    gRNA of DRA-43


<400>    210                                                                    20
ugcaccuugagccucaaagc


<210>    211
<211>    20
<212>    RNA
<213>    Artificial Sequence

<220>
<223>    gRNA of DRA-44


<400>    211                                                                    20
ugcauuggccaacauagcug


<210>    212
<211>    20
<212>    RNA
<213>    Artificial Sequence

<220>
<223>    gRNA of DRA-45


<400>    212                                                                    20
uggacgauuugccagcuuug


<210>    213
<211>    20
<212>    RNA
<213>    Artificial Sequence

<220>
<223>    gRNA of DRA-46

<400>    213                                                                    20
uggcaaagaaggagacgguc

<210>    214
<211>    20
<212>    RNA
<213>    Artificial Sequence

<220>
<223>    gRNA of DRA-47

<400>    214                                                                    20
uggugaugagauuuuccaug

<210>    215
<211>    20
<212>    RNA
<213>    Artificial Sequence

<220>
<223>    gRNA of DRA-48

<400>    215                                                                    20
aaugucacguggcuucgaaa

<210>    216
<211>    20
<212>    RNA
<213>    Artificial Sequence

<220>
<223>    gRNA of DRA-49

<400>     216                                                                   20
agacaaguucaccccaccag

<210>    217
<211>    20
<212>    RNA
<213>    Artificial Sequence

<220>
<223>    gRNA of DRA-50

<400>    217                                                                    20
agacaaguucaccccaccag

<210>    218
<211>    20
<212>    RNA

<213>    Artificial Sequence

<220>
<223>    gRNA of DRA-51


<400>    218                                                                    20
gaacgcagggggccucugua


<210>    219
<211>    20
<212>    RNA
<213>    Artificial Sequence

<220>
<223>    gRNA of DRA-52


<400>    219                                                                    20
cugaggacguuuacgacugc


<210>    220
<211>    20
<212>    RNA
<213>    Artificial Sequence

<220>
<223>    gRNA of DRA-53


<400>    220                                                                    20
gcggaaaaggugggucuuccc


<210>    221
<211>    20
<212>    RNA
<213>    Artificial Sequence

<220>
<223>    gRNA of DRA-54


<400>    221                                                                    20
ggacguuuacgacugcaggg


<210>    222
<211>    20
<212>    RNA
<213>    Artificial Sequence

<220>
<223>    gRNA of DRA-55


<400>    222                                                                    20
gucguaaacguccucaguug


<210>    223

<211>    20
<212>    RNA
<213>    Artificial Sequence

<220>
<223>    gRNA of DRA-56


<400>    223                                                          20
gugagcacaguuaccucugg


<210>    224
<211>    20
<212>    RNA
<213>    Artificial Sequence

<220>
<223>    gRNA of DRA-57


<400>    224                                                          20
guguccccaguaccuccag


<210>    225
<211>    20
<212>    RNA
<213>    Artificial Sequence

<220>
<223>    gRNA of DRA-58


<400>    225                                                          20
ugaggacguuuacgacugca


<210>    226
<211>    20
<212>    RNA
<213>    Artificial Sequence

<220>
<223>    gRNA of DRA-59


<400>    226                                                          20
aauggaaaaccugucaccac


<210>    227
<211>    20
<212>    RNA
<213>    Artificial Sequence

<220>
<223>    gRNA of DRA-60


<400>    227                                                          20
aguggaacuugcggaaaagg

<210>    228
<211>    20
<212>    RNA
<213>    Artificial Sequence

<220>
<223>    gRNA of DRA-61


<400>    228                                                                    20
augaaacagaugaggacguu


<210>    229
<211>    20
<212>    RNA
<213>    Artificial Sequence

<220>
<223>    gRNA of DRA-62


<400>    229                                                                    20
cagagacagucuuccugccc


<210>    230
<211>    20
<212>    RNA
<213>    Artificial Sequence

<220>
<223>    gRNA of DRA-63


<400>    230                                                                    20
cgugacauugaccacuggug


<210>    231
<211>    20
<212>    RNA
<213>    Artificial Sequence

<220>
<223>    gRNA of DRA-64


<400>    231                                                                    20
uaugaaacagaugaggacgu


<210>    232
<211>    20
<212>    RNA
<213>    Artificial Sequence

<220>
<223>    gRNA of DRA-65


<400>    232                                                                    20

ucugacacuccuguggugac

<210>    233
<211>    20
<212>    RNA
<213>    Artificial Sequence

<220>
<223>    gRNA of DRA-66

<400>    233                                                              20
aaacguccucaguugagggc

<210>    234
<211>    20
<212>    RNA
<213>    Artificial Sequence

<220>
<223>    gRNA of DRA-67

<400>    234                                                              20
ucguaaacguccucaguuga

<210>    235
<211>    20
<212>    PRT
<213>    Artificial Sequence

<220>
<223>    B2M signal peptide

<400>    235
Met Ser Arg Ser Val Ala Leu Ala Val Leu Ala Leu Leu Ser Leu Ser
 1               5                   10                  15

Gly Leu Glu Ala
            20

<210>    236
<211>    9
<212>    PRT
<213>    Artificial Sequence

<220>
<223>    G-peptide

<400>    236
Val Met Ala Pro Arg Thr Leu Phe Leu
 1               5

<210>    237
<211>    99
<212>    PRT

<213>    Artificial Sequence

<220>
<223>    B2M

<400>    237

<210>    238
<211>    15
<212>    PRT
<213>    Artificial Sequence

<220>
<223>    G4S linker 1

<400>    238
Gly Gly Gly Gly Ser Gly Gly Gly Gly Ser Gly Gly Gly Gly Ser
1               5                   10                  15

<210>    239
<211>    9
<212>    PRT
<213>    Artificial Sequence

<220>
<223>    HA tag

<400>    239
Tyr Pro Tyr Asp Val Pro Asp Tyr Ala
1               5

<210>    240
<211>    337
<212>    PRT
<213>    Artificial Sequence

<220>

<223>    HLA-E

<400>    240
Gly Ser His Ser Leu Lys Tyr Phe His Thr Ser Val Ser Arg Pro Gly
1               5                   10                  15

Arg Gly Glu Pro Arg Phe Ile Ser Val Gly Tyr Val Asp Asp Thr Gln
            20                  25                  30

Phe Val Arg Phe Asp Asn Asp Ala Ala Ser Pro Arg Met Val Pro Arg
        35                  40                  45

Ala Pro Trp Met Glu Gln Glu Gly Ser Glu Tyr Trp Asp Arg Glu Thr
    50                  55                  60

Arg Ser Ala Arg Asp Thr Ala Gln Ile Phe Arg Val Asn Leu Arg Thr
65                  70                  75                  80

Leu Arg Gly Tyr Tyr Asn Gln Ser Glu Ala Gly Ser His Thr Leu Gln
                85                  90                  95

Trp Met His Gly Cys Glu Leu Gly Pro Asp Gly Arg Phe Leu Arg Gly
            100                 105                 110

Tyr Glu Gln Phe Ala Tyr Asp Gly Lys Asp Tyr Leu Thr Leu Asn Glu
        115                 120                 125

Asp Leu Arg Ser Trp Thr Ala Val Asp Thr Ala Ala Gln Ile Ser Glu
    130                 135                 140

Gln Lys Ser Asn Asp Ala Ser Glu Ala Glu His Gln Arg Ala Tyr Leu
145                 150                 155                 160

Glu Asp Thr Cys Val Glu Trp Leu His Lys Tyr Leu Glu Lys Gly Lys
                165                 170                 175

Glu Thr Leu Leu His Leu Glu Pro Pro Lys Thr His Val Thr His His
            180                 185                 190

Pro Ile Ser Asp His Glu Ala Thr Leu Arg Cys Trp Ala Leu Gly Phe
        195                 200                 205

Tyr Pro Ala Glu Ile Thr Leu Thr Trp Gln Gln Asp Gly Glu Gly His
    210                 215                 220

Thr Gln Asp Thr Glu Leu Val Glu Thr Arg Pro Ala Gly Asp Gly Thr
225                 230                 235                 240

Phe Gln Lys Trp Ala Ala Val Val Val Pro Ser Gly Glu Glu Gln Arg
                245                 250                 255

Tyr Thr Cys His Val Gln His Glu Gly Leu Pro Glu Pro Val Thr Leu
            260                 265                 270

Arg Trp Lys Pro Ala Ser Gln Pro Thr Ile Pro Ile Val Gly Ile Ile
        275                 280                 285

Ala Gly Leu Val Leu Leu Gly Ser Val Val Ser Gly Ala Val Val Ala
    290                 295                 300

Ala Val Ile Trp Arg Lys Lys Ser Ser Gly Gly Lys Gly Gly Ser Tyr
305                 310                 315                 320

Ser Lys Ala Glu Trp Ser Asp Ser Ala Gln Gly Ser Glu Ser His Ser
                325                 330                 335

Leu

<210>    241
<211>    20
<212>    PRT
<213>    Artificial Sequence

<220>
<223>    G4S linker 2

<400>    241
Gly Gly Gly Gly Ser Gly Gly Gly Gly Ser Gly Gly Gly Gly Ser Gly
1               5                   10                  15

Gly Gly Gly Ser
            20

<210>    242
<211>    20
<212>    DNA
<213>    Artificial Sequence

<220>
<223>    forward primer

<400>    242
ctggcttgga gacaggtgac                                                   20

<210>    243
<211>    20
<212>    DNA
<213>    Artificial Sequence

<220>
<223>    reverse primer

<400>    243
gacgcttatc gacgccctaa                                                   20

<210>    244
<211>    21
<212>    DNA
<213>    Artificial Sequence

<220>
<223>    forward primer

<400>    244
cccaagtgaa ataccctggc a                                                 21

<210>    245
<211>    20
<212>    DNA
<213>    Artificial Sequence

<220>
<223>    reverse primer


<400>    245
agcccttcct actagcctca                                                    20


<210>    246
<211>    20
<212>    DNA
<213>    Artificial Sequence

<220>
<223>    forward primer


<400>    246
aatttcttgg ggagggggtg                                                    20


<210>    247
<211>    24
<212>    DNA
<213>    Artificial Sequence

<220>
<223>    reverse primer


<400>    247
agctggatag taggagaaga cagt                                               24


<210>    248
<211>    22
<212>    DNA
<213>    Artificial Sequence

<220>
<223>    forward primer


<400>    248
gggttaaaga gtctgtccgt ga                                                 22


<210>    249
<211>    23
<212>    DNA
<213>    Artificial Sequence

<220>
<223>    reverse primer


<400>    249

tgtcgagacc acataatacc tgt                                                23

<210>    250
<211>    20
<212>    DNA
<213>    Artificial Sequence

<220>
<223>    forward primer

<400>    250
acctgacttg gcagggtttg                                                    20

<210>    251
<211>    21
<212>    DNA
<213>    Artificial Sequence

<220>
<223>    reverse primer

<400>    251
cccaagatct accaccggag a                                                  21

<210>    252
<211>    20
<212>    DNA
<213>    Artificial Sequence

<220>
<223>    forward primer

<400>    252
tgctcccaag cagaaggtaa                                                    20

<210>    253
<211>    24
<212>    DNA
<213>    Artificial Sequence

<220>
<223>    reverse primer

<400>    253
aacccatgaa gtgtggaaaa caag                                               24

<210>    254
<211>    20
<212>    DNA
<213>    Artificial Sequence

<220>
<223>    forward primer

<400>    254
tccctccata ccagggttca                                                    20


<210>    255
<211>    21
<212>    DNA
<213>    Artificial Sequence

<220>
<223>    reverse primer


<400>    255
aactcatcct taccccagtg t                                                  21


<210>    256
<211>    20
<212>    DNA
<213>    Artificial Sequence

<220>
<223>    forward primer


<400>    256
tgtgtcaact tatgccgcgt                                                    20


<210>    257
<211>    20
<212>    DNA
<213>    Artificial Sequence

<220>
<223>    reverse primer


<400>    257
ttgggaaaca cggtcacctc                                                    20


<210>    258
<211>    21
<212>    DNA
<213>    Artificial Sequence

<220>
<223>    forward primer


<400>    258
tgtgaactgg agctctcttg a                                                  21


<210>    259
<211>    21
<212>    DNA
<213>    Artificial Sequence

<220>

<223>    reverse primer

<400>    259
tatgagggcc agagggaaca t                                                21